(19) Europäisches Patentamt

European Patent Office

Office européen des brevets

(11) **EP 2 119 454 A1**

(12) **EUROPEAN PATENT APPLICATION**
published in accordance with Art. 153(4) EPC

(43) Date of publication:
**18.11.2009  Bulletin 2009/47**

(21) Application number: **07860086.3**

(22) Date of filing: **25.12.2007**

(51) Int Cl.:
*A61K 47/38* (2006.01)   *A61K 9/20* (2006.01)
*A61K 31/795* (2006.01)   *A61K 47/02* (2006.01)
*A61K 47/10* (2006.01)   *A61K 47/26* (2006.01)
*A61K 47/32* (2006.01)   *A61K 47/44* (2006.01)

(86) International application number:
**PCT/JP2007/074857**

(87) International publication number:
**WO 2008/084676 (17.07.2008 Gazette 2008/29)**

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **26.12.2006  JP 2006349401**

(71) Applicant: **Medrx Co., Ltd.
Higashikagawa-shi
Kagawa 769-2712 (JP)**

(72) Inventors:
• **ISHIBASHI, Masaki
Higashikagawa-shi
Kagawa 769-2712 (JP)**

• **KOBAYASHI, Katsunori
Higashikagawa-shi
Kagawa 769-2712 (JP)**
• **HAMAMOTO, Hidetoshi
Higashikagawa-shi
Kagawa 769-2712 (JP)**

(74) Representative: **Duckworth, Timothy John
J.A. Kemp & Co.,
14 South Square,
Gray's Inn
London WC1R 5JJ (GB)**

(54) **PHARMACEUTICAL COMPOSITION COMPRISING POROUS DRY MATRIX**

(57)     A new administration form for oral administration is provided.

A porous dry matrix preparation comprising at least a polymeric thickener and an excipient ingredient, wherein
(1) the content of the polymeric thickener is down to 0.5 w/w% and up to 14 w/w%,
(2) the content of the excipient ingredient is down to 30 w/w% and up to 80 w/w%,
(3) the void ratio is not less than 20%, and
(4) the water activity is not more than 0.55, or water content is not more than 10 w/w%.

EP 2 119 454 A1

**Description**

Technical Field

The present invention relates to an oral pharmaceutical composition, which contains a drug in a porous dry matrix comprising at least a polymeric thickener (carboxymethylcellulose salt etc.) and an excipient. Furthermore, the present invention relates to a production method of the oral pharmaceutical composition.

Background Art

When a pharmaceutical product is administered orally, solid preparations such as tablet, granule, powder and the like are generally used. Recently, the development of new drug delivery systems (DDSs) is ongoing, and investigation on jelly preparations, among others, has been undertaken. That is, jelly preparation has been widely studied as a method of developing a new dosage form, since it has high water content and is superior in the impression on ingestion, it can easily avoid the risk of accidental ingestion in aged patients, and the like.
Jelly preparation is **characterized in that** it permits slow-release of a drug and improvement of impression on ingestion by dissolving or dispersing an active ingredient along with water in a gel matrix. For example, patent reference 1 discloses a pharmaceutical jelly composition mainly containing carageenan, which generally shows dissolution property equivalent to or higher than that of tablet, powder and the like for oral administration, as well as superior preservation stability.

However, high water content as that of jelly preparation often seriously influences the stability of the active ingredient. To be specific, an active ingredient susceptible to hydrolysis cannot be formulated into a jelly preparation with ease, which in turn limits the applicable range of the drug. Moreover, since jelly preparation has a larger packaging size and a greater weight as compared to general tablets, when patients receive jelly preparations in the amount of administration corresponding to the necessary number of days at one time in a pharmacy or carry them in daily life, they are problematically faced with inconvenience as compared to tablets.

Hence, in order to improve the convenience for patients, for example, patent reference 2 discloses production of a dehydrated jelly product well restorable in water, by freeze-drying a jelly preparation containing xanthan gum. Additionally, it is indicated that this dehydrated jelly product shows improved long-term preservation stability, and improved convenience for patients because of its easy administration by portions.

However, for mass production of the dehydrated jelly products by freeze-drying a jelly preparation, there remain problems in terms of apparatus and cost, and further modification is considered to be necessary.
While patent reference 2 discloses re-impregnating a dehydrated jelly product with water and eating the jelly, the water re-impregnation dehydrated jelly agent disclosed therein is a crash-jelly rather than a molded block of jelly, and is not very different from liquid agents in the handlability during administration. Accordingly, the jelly agent after water re-impregnation of patent reference 2 is unable to utilize the advantage of general jelly preparations.

On the other hand, patent reference 3 discloses a wafery dosage form which is rapidly disintegrated in the oral cavity, and a polymer matrix using polyvinyl alcohol is described in the production examples.
However, this wafery product is characterized by easy disintegration in water, and cannot be re-impregnated with water. Furthermore, only polyvinyl alcohol is disclosed as a material of the preparation, and the reference is completely silent on carboxymethylcellulose salt.

Carboxymethylcellulose salt, which is used as a base material of a preparation in the present invention, is often used as a disintegrant in oral solid preparations (tablet and the like) in the field of pharmaceutical products. In this case, however, the carboxymethylcellulose salt functions to afford disintegration property of a tablet in water or stomach fluid. Accordingly, there has not been known at all heretofore that a porous dry matrix can be constructed using carboxymethylcellulose salt as a skeletal material, and that a water-containing matrix, which is stable even when immersed in water, can be prepared. Moreover, applicability of the porous dry matrix and a preparation thereof as oral preparations has not been known at all heretofore.

In addition, a preparation of polystyrene sulfonate, which is one of the objects of the present invention, has been conventionally used as a therapeutic agent for hypercalcemia in renal failure patients, and 15 to 30 g of polystyrene sulfonate is generally administered to an adult per day. However, since polystyrene sulfonate is easily precipitated at the bottom of a cup even when suspended in water, a single administration of the entire amount thereof is difficult. Moreover, since polystyrene sulfonate requires high dose and is highly uncomfortable when contained in the mouth, it is a pharmaceutical agent difficult to take for the patients. In order to improve this, attempts have been made to improve the impression on ingestion and the amount of water by forming a gel-like preparation (jelly preparation). However, there remain many problems yet to be improved since it has been clarified that the drug efficacy varies depending on the manner of destruction of the gel during administration (Yakuzaigaku, 60(4), 261-270 (2000)).

Patent Reference 1: JP-A-2004-99559

# EP 2 119 454 A1

Patent Reference 2: JP-A-2004-97114
Patent Reference 3: Japanese Patent Application 2004-501958
Non-patent Reference 1: Yakuri-To-Chiryo, Vol.21, No.6. 379 (1993)

Disclosure of the Invention

Problems to be Solved by the Invention

It is an object of the present invention to provide a new porous dry matrix, and a new porous dry matrix preparation comprising various drugs and reagents included and impregnated therein. It is another object of the present invention to provide a method of producing a new porous dry matrix, which produces the porous dry matrix by a heat treatment alone, rather than freeze-drying.

Means for Solving the Problems

The present inventors investigated the preparation of a porous dry matrix (xerogel) and applications thereof in order to construct a new preparation system. Conventionally, it has been general practice for preparing an orally ingestible porous dry matrix to first prepare jellies, and freeze-dry the same to yield a porous dry matrix, as disclosed in patent document 2. However, since many problems remain to be solved to achieve mass-production of the finished product, the present inventors diligently investigated production of a porous dry matrix by a method other than freeze-drying, i.e., thermal drying treatment under normal pressure.

As a result, the present inventors could find that the new porous dry matrix obtained offers a cookie-like impression on ingestion when taken in a dry state; furthermore, for example, in the case of elderly people with weak teeth having difficulty in taking a preparation in a dry state, the new porous dry matrix quickly absorbs water and becomes a jelly-like composition that is unlikely to collapse and offers a good impression on ingestion when impregnated in water. Furthermore, during that investigation, it was demonstrated that to prepare the porous dry matrix of the present invention and a porous dry matrix preparation prepared using the same (hereinafter, both are referred to as porous dry matrix preparations irrespective of the presence or absence of a pharmacologically effective ingredient unless specially distinguished), the following two factors are important.

(1) Structural factors for porous dry matrix preparation:

To prepare a porous dry matrix preparation that is orally ingestible and has good eating quality, it is suitable that the content of the structure ingredient and excipient ingredient described below fall in the range from about 15% to 60% before drying, and the quantitative ratio of the structure ingredient and excipient ingredient is in the range from about 3 to 100 (relative to the structure ingredient as 1).

1. Structure ingredient: Polymeric thickeners can be used.

For example, cellulose derivatives such as carboxymethylcellulose salt and polysaccharide thickeners such as guar gum and carrageenan can be used.

2. Excipient ingredient: A powder that is almost insoluble in water can be used.

(2) Factors for improving the impression on ingestion of porous dry matrix preparation:

If mainly the eating quality of an excipient ingredient is poor, an improvement in impression on ingestion can be achieved by the following measures:

1. Improvement of tooth touch

Tooth touch can be improved by assuring a void ratio of not less than about 20% and using a sugar and/or a sugar alcohol.
To assure a void ratio of about 20%, the water content of the mixed composition before drying is suitably not less than about 25%.

2. Improvement of tongue touch (flouriness):

For obtaining a masking effect, an edible oil/fat can be used.

3. Improvement of flavor:

To help ingestion, to impart a flavor directly to a porous dry matrix preparation, or to mask the bitterness of the drug contained therein and the like, for example, a sweetener, a pH regulator, a flavoring agent and the like can be chosen according to the intended use.

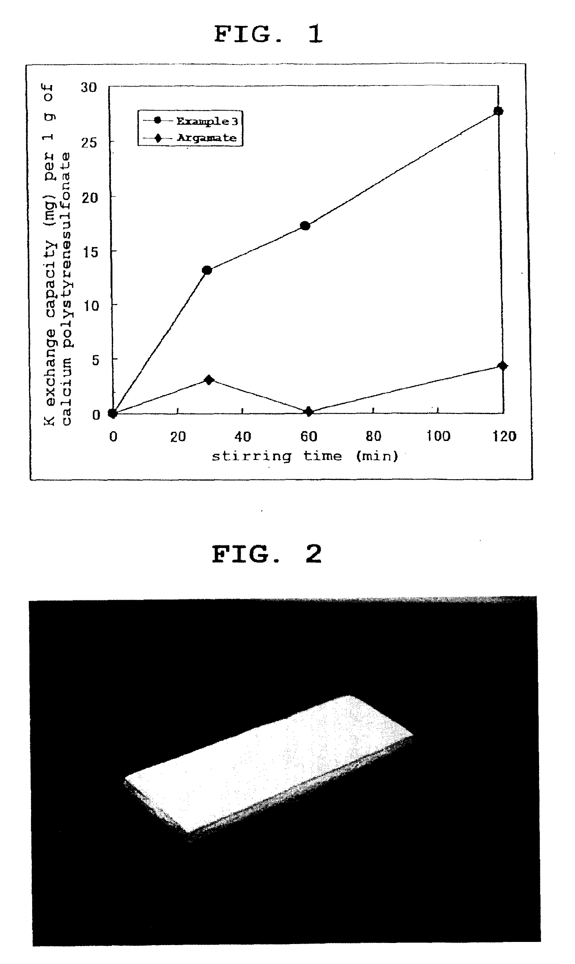

As a method of producing a porous dry matrix preparation, taking the foregoing two factors into consideration, a specified structure ingredient (i.e., polymeric thickener) and an excipient ingredient are blended in water, and the mixture is kneaded to a clay-like form. This is molded and thermally dried, and drying treatment is performed until the water activity becomes not more than 0.60, preferably not more than 0.55, or until the water content becomes not more than 10%, preferably not more than 8%. Thereby, a porous dry matrix preparation as shown in FIG. 2 can be prepared. The present inventors found that in this case, if various drugs and reagents are co-present, they are incorporated in the matrix formed during thermal drying treatment, and become a porous dry matrix preparation containing a desired pharmacologically effective ingredient after drying.
Furthermore, the present inventors also found that a new desired porous dry matrix preparation can be prepared by preparing a initial porous dry matrix not containing a drug, but after completion of drying and production, impregnating the porous dry matrix with a drug dissolved in water or oil, and drying the same.
These porous dry matrix preparations have well-masked discomfort derived mainly from pharmacologically effective ingredient and excipient ingredient, and can be taken as cookie-like or biscuit-like preparations while in a dry state.

The present inventors conducted further investigations, and found that particularly when carboxymethylcellulose salt is used as the structure ingredient, the porous dry matrix preparation of the present invention, when impregnated in water, absorbs water in a short time to yield a finely-textured, sponge-like wet preparation. This wet preparation retains the preparation strength such that it can be held up by fingertips. It was found that this wet preparation has a jelly-like touch, and offers a better impression on ingestion than jelly preparations.

The present inventors developed the present invention based on these findings.

Accordingly, the present invention provides the following:

[1] A porous dry matrix preparation comprising at least a polymeric thickener and an excipient ingredient, wherein

    (1) the content of the polymeric thickener is down to 0.5 w/w% and up to 14 w/w%,
    (2) the content of the excipient ingredient is down to 30 w/w% and up to 80 w/w%,
    (3) the void ratio is not less than 20%, and
    (4) the water activity is not more than 0.55, or water content is not more than 10 w/w%.

Furthermore, the preparation is preferably one wherein (5) the ratio by weight of the excipient ingredient to the polymeric thickener is about 3 to 100.
[2] The preparation of [1] above, wherein the polymeric thickener comprises carboxymethylcellulose and/or a salt thereof.
[3] The preparation of [2] above, wherein the carboxymethylcellulose salt is carboxymethylcellulose sodium or carboxymethylcellulose calcium.
[4] The preparation of any of [1] to [3] above, wherein the excipient ingredient is a powder that is almost insoluble in water.
[5] The preparation of [4] above, wherein the excipient ingredient being the powder that is almost insoluble in water is one kind or a mixture of two or more kinds selected from the group consisting of cellulose-series polymers, starch, synthetic polymers, and activated charcoal.
[6] The preparation of [5] above, wherein the cellulose-series polymer is crystalline cellulose.
[7] The preparation of any of [1] to [6] above, comprising an edible oil/fat.
[8] The preparation of any of [1] to [7] above, comprising a sugar and/or a sugar alcohol.
[9] The preparation of [8] above, wherein the sugar and/or a sugar alcohol is one kind or a mixture of two or more kinds selected from the group consisting of white sugar, powdered reduced maltose syrup, sorbitol, and mannitol.
[10] The preparation of any of [1] to [9] above, comprising a pharmacologically effective ingredient.

[11] A porous dry matrix preparation comprising at least polymeric thickener and a polystyrenesulfonate, wherein

(1) the content of the polymeric thickener is down to 0.5 w/w% and up to 14 w/w%,
(2) the content of the polystyrenesulfonate is down to 30 w/w% and up to 80 w/w%,
(3) the void ratio is not less than 20%, and
(4) the water activity is not more than 0.55, or water content is not more than 10 w/w%.

Furthermore, the preparation is preferably one wherein (5) the ratio by weight of the excipient ingredient to the polymeric thickener is about 3 to 100.

[12] The preparation of [11] above, wherein the polymeric thickener comprises carboxymethylcellulose and/or a salt thereof.

[13] The preparation of [12] above, wherein the carboxymethylcellulose salt is carboxymethylcellulose sodium or carboxymethylcellulose calcium.

[14] The preparation of any of [11] to [13] above, wherein the polystyrenesulfonate is calcium polystyrenesulfonate or sodium polystyrenesulfonate.

[15] The preparation of any of [11] to [14] above, comprising an edible oil/fat.

[16] The preparation of any of [11] to [15] above, comprising a sugar and/or a sugar alcohol.

[17] The preparation of [16] above, wherein the sugar and/or the sugar alcohol is one kind or a mixture of two or more kinds selected from the group consisting of white sugar, powdered reduced maltose syrup, sorbitol, and mannitol.

[18] The preparation of any of [11] to [17] above, comprising a pharmacologically effective ingredient.

[19] The preparation of [18] above, wherein the polystyrenesulfonate is also a pharmacologically effective ingredient.

The present invention also provides the following.

[20] A porous dry matrix preparation comprising at least carboxymethylcellulose sodium, calcium polystyrenesulfonate, a sugar and/or a sugar alcohol and an edible oil/fat, wherein

(1) the content of the carboxymethylcellulose sodium is down to 0.5 w/w% and up to 14 w/w%,
(2) the content of the calcium polystyrenesulfonate is down to 30 w/w% and up to 80 w/w%,
(3) the void ratio is not less than 20%, and
(4) the water activity is not more than 0.55, or water content is not more than 10 w/w%.

Furthermore, the preparation is preferably one wherein (5) the ratio by weight of the excipient ingredient to the polymeric thickener is about 3 to 100.

[21] The preparation of [20] above, wherein the sugar and/or the sugar alcohol is one kind or a mixture of two or more kinds selected from the group consisting of white sugar, powdered reduced maltose syrup, sorbitol, and mannitol.

The present invention further provides the following.

[22] A porous dry matrix preparation comprising at least polymeric thickener and an excipient ingredient, wherein

(1) the content of the polymeric thickener is down to 0.5 w/w% and up to 14 w/w%,
(2) the content of the excipient ingredient is down to 30 w/w% and up to 80 w/w%,
(3) the void ratio is not less than 20%, and
(4) the water activity is not more than 0.55, or water content is not more than 10 w/w%,

and, furthermore, wherein the preparation completes water absorption within 15 minutes to become a hydrated composition that does not collapse when impregnated with water. Furthermore, the preparation is preferably one wherein (5) the ratio by weight of the excipient ingredient to the polymeric thickener is about 5 to 100.

[23] The preparation of [22] above, wherein the polymeric thickener comprises carboxymethylcellulose and/or a salt thereof.

[24] The preparation of [23] above, wherein the carboxymethylcellulose salt is carboxymethylcellulose sodium or carboxymethylcellulose calcium.

[25] The preparation of any of [22] to [24] above, wherein the excipient ingredient is a powder that is almost insoluble in water.

[26] The preparation of [25] above, wherein the excipient ingredient being the powder that is almost insoluble in water is one kind or a mixture of two or more kinds selected from the group consisting of cellulose-series polymers, starch, synthetic polymers, and activated charcoal.

[27] The preparation of [26] above, wherein the cellulose-series polymer is crystalline cellulose.

[28] The preparation of any of [22] to [27] above, comprising an edible oil/fat.

[29] The preparation of any of [22] to [28] above, comprising a sugar and/or a sugar alcohol.

[30] The preparation of [29] above, wherein the sugar and/or the sugar alcohol is one kind or a mixture of two or more kinds selected from the group consisting of white sugar, powdered reduced maltose syrup, sorbitol, and mannitol.

[31] The preparation of any of [22] to [30] above, comprising a pharmacologically effective ingredient.

[32] A porous dry matrix preparation comprising at least a polymeric thickener and polystyrenesulfonate, wherein

(1) the content of the polymeric thickener is down to 0.5 w/w% and up to 14 w/w%,

(2) the content of the polystyrenesulfonate is down to 30 w/w% and up to 80 w/w%,

(3) the void ratio is not less than 20%, and

(4) the water activity is not more than 0.55, or water content is not more than 10 w/w%,

and, furthermore, wherein the preparation completes water absorption within 15 minutes to become a hydrated composition that does not collapse when impregnated with water. Furthermore, the preparation is preferably one wherein (5) the ratio by weight of the excipient ingredient to the polymeric thickener is about 5 to 100.

[33] The preparation of [32] above, wherein the polymeric thickener comprises carboxymethylcellulose and/or a salt thereof.

[34] The preparation of [33] above, wherein the carboxymethylcellulose salt is carboxymethylcellulose sodium or carboxymethylcellulose calcium.

[35] The preparation of any of [32] to [34] above, wherein the polystyrenesulfonate is calcium polystyrenesulfonate or sodium polystyrenesulfonate.

[36] The preparation of any of [32] to [35] above, comprising an edible oil/fat.

[37] The preparation of any of [32] to [36] above, comprising a sugar and/or a sugar alcohol.

[38] The preparation of [37] above, wherein the sugar and/or the sugar alcohol is one kind or a mixture of two or more kinds selected from the group consisting of white sugar, powdered reduced maltose syrup, sorbitol, and mannitol.

[39] The preparation of any of [32] to [38] above, comprising a pharmacologically effective ingredient.

[40] The preparation of [39] above, wherein the polystyrenesulfonate is also a pharmacologically effective ingredient.

The present invention further provides the methods of production described below.

[41] A method of producing the porous dry matrix preparation of any of [1] to [10] above, comprising subjecting a mixed composition obtained by mixing and molding at least a polymeric thickener, an excipient ingredient, and water to thermal drying under normal pressure, wherein the total content of the polymeric thickener and the excipient ingredient is 15 w/w% to 60 w/w%, and wherein the water content before thermal drying is not less than 25 w/w%.

[42] The method of [41] above, wherein the thermal drying under normal pressure is performed at a temperature of 100˚C to 200˚C.

[43] The method of [41] above, wherein the thermal drying under normal pressure is performed at a temperature of 140˚C to 150˚C or more after thermal drying is performed at a temperature of 60˚C to 130˚C.

[44] A method of producing the porous dry matrix preparation of any of [11] to [19] above, comprising subjecting a mixed composition obtained by mixing and molding at least a polymeric thickener, polystyrenesulfonate, and water to thermal drying under normal pressure, wherein the total content of the polymeric thickener and the polystyrenesulfonate is 15 w/w% to 60 w/w%, and wherein the water content before thermal drying is not less than 25 w/w%.

[45] The method of [44] above, wherein the thermal drying under normal pressure is performed at a temperature of 100˚C to 200˚C.

[46] The method of [44] above, wherein the thermal drying under normal pressure is performed at a temperature of 140˚C to 150˚C or more after thermal drying is performed at a temperature of 60˚C to 130˚C.

[47] A method of producing the porous dry matrix preparation of any of [22] to [31] above, comprising subjecting a mixed composition obtained by mixing and molding at least a polymeric thickener, an excipient ingredient, and water to thermal drying under normal pressure, wherein the total content of the polymeric thickener and the excipient ingredient is 15 w/w% to 60 w/w%, and wherein the water content before thermal drying is not less than 25 w/w%.

[48] The method of [47] above, wherein the thermal drying under normal pressure is performed at a temperature of 100˚C to 200˚C.

[49] The method of [47] above, wherein the thermal drying under normal pressure is performed at a temperature of 140˚C to 150˚C or more after thermal drying is performed at a temperature of 60˚C to 130˚C.

[50] A method of producing the porous dry matrix preparation of any of [32] to [40] above, comprising subjecting a mixed composition obtained by mixing and molding at least a polymeric thickener, polystyrenesulfonate, and water to thermal drying under normal pressure, wherein the total content of the polymeric thickener and the polystyrenesulfonate is 15 w/w% to 60 w/w%, and wherein the water content before thermal drying is not less than 25 w/w%.

[51] The method of [50] above, wherein the thermal drying under normal pressure is performed at a temperature of 100˚C to 200˚C.

[52] The method of [50] above, wherein the thermal drying under normal pressure is performed at a temperature of 140˚C to 150˚C or more after thermal drying is performed at a temperature of 60˚C to 130˚C.

[53] A method of producing the porous dry matrix preparation of [10] above, comprising subjecting a mixed composition obtained by mixing and molding at least a polymeric thickener, an excipient ingredient, and water to thermal drying under normal pressure to yield a porous dry matrix composition, wherein the total content of the polymeric thickener and the excipient ingredient is 15 w/w% to 60 w/w%, and wherein the water content before thermal drying is not less than 25 w/w%, and
impregnating the dry matrix composition with a solution of a pharmacologically effective ingredient, and, as required, evaporating the solvent for the solution.

[54] The method of [53] above, wherein the thermal drying under normal pressure is performed at a temperature of 100˚C to 200˚C.

[55] The method of [53] above, wherein the thermal drying under normal pressure is performed at a temperature of 140˚C to 150˚C or more after thermal drying is performed at a temperature of 60˚C to 130˚C.

[56] A method of producing the porous dry matrix preparation of [31] above, comprising subjecting a mixed composition obtained by mixing and molding at least a polymeric thickener, an excipient ingredient, and water to thermal drying under normal pressure to yield a porous dry matrix composition, wherein the total content of the polymeric thickener and the excipient ingredient is 15 w/w% to 60 w/w%, and wherein the water content before thermal drying is not less than 25 w/w%, and
impregnating the dry matrix composition with a solution of a pharmacologically effective ingredient, and, as required, evaporating the solvent for the solution.

[57] The method of [56] above, wherein the thermal drying under normal pressure is performed at a temperature of 100˚C to 200˚C.

[58] The method of [56] above, wherein the thermal drying under normal pressure is performed at a temperature of 140˚C to 150˚C or more after thermal drying is performed at a temperature of 60˚C to 130˚C.

Effect of the Invention

In the present invention, as a result of the finding of a new porous dry matrix composition, it has become possible to prepare a new desired porous dry matrix preparation wherein various drugs and reagents are included and impregnated in the space of the matrix. As a result, because even a drug and reagent with a poor impression on ingestion can be incorporated in a porous dry matrix, good impression on ingestion is obtained even if a drug-including porous dry matrix preparation is taken as is. Hence, in the present invention, a porous dry matrix preparation with an improved impression on ingestion can be provided.
Because the porous dry matrix preparation of the present invention assumes a dry dosage form, the preparation has better drug storage stability and reduced weights compared with jelly preparations, which facilitates mobile use. Furthermore, by adding an additive such as a sweetener, a dry preparation with a further improved impression on ingestion and better ingestibility can be provided.

Particularly, the polystyrenesulfonate-containing porous dry matrix preparation of the present invention is a new, more effective dosage form that is easily ingestible by renal insufficiency patients under limitations on water intake. Additionally, this preparation has significantly improved features for convenience for patients, including the absence of an influence of preparation shape, size and the like on potassium exchange rate (capacity), and significantly reduced weights compared with gel-like preparations, which facilitates mobile use.
Furthermore, the porous dry matrix preparation of the present invention absorbs water in a short time to become a wet preparation when impregnated in water. This wet preparation offers an impression on ingestion better than that of jelly preparations, and became applicable to many pharmaceuticals as a new means of preparation making.

Brief Description of the Drawings

[FIG. 1] FIG. 1 is a graph showing the results of a potassium exchange capacity comparative test of a porous dry matrix preparation of calcium polystyrenesulfonate and a commercially available calcium polystyrenesulfonate salt jelly (Argamate jelly) .

[FIG. 2] FIG. 2 is a photograph of the external appearance of the calcium polystyrenesulfonate-containing preparation of the present invention.

[FIG. 3] FIG. 3 is a cross-sectional photograph of the calcium polystyrenesulfonate-containing preparation of the present invention (photograph showing the presence of fine pores). Best Mode for Carrying out the Invention

- First aspect of the present invention -

A first aspect of the present invention relates to a porous dry matrix preparation.

As mentioned herein, "porous dry matrix preparation" means a porous dry gel (xerogel) composition comprising at least a polymeric thickener, which is a structure ingredient, and an excipient ingredient. As is obvious to those skilled in the art, dry gel usually has a large number of pores in the structure thereof, and as mentioned herein, the term "porous" refers to a state wherein the "void ratio" as described below is normally not less than 20%, preferably not less than 25%, more preferably not less than 30%, and not more than 70%. Therefore, as mentioned herein, a "porous dry matrix preparation" normally means a dry gel composition comprising at least a polymeric thickener and an excipient ingredient having a void ratio of not less than 20%. Drying refers to, for example, a state wherein the water content is not more than 10%.

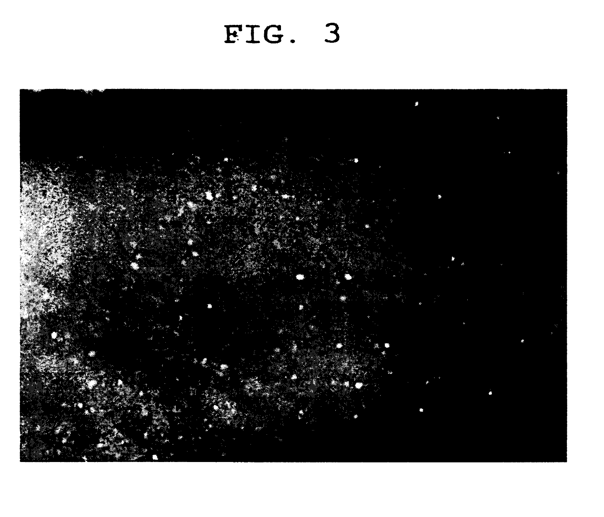

An example of the porous dry matrix preparation of the present invention is shown in FIG. 2. An example cross-sectional view of the porous dry matrix preparation is shown in FIG. 3. In the example of FIG. 3, a large number of fine pores dispersed are observed in a cross section of the preparation.

Regarding the shape of the porous dry matrix preparation of the present invention, the preparation can be prepared in an optionally chosen shape, including rectangular parallelepipeds, discs, doughnuts, grains and the like, according to the intended use as appropriate.

As mentioned herein, a "polymeric thickener" means a polymeric substance that is soluble or dispersible in water to increase viscosity, and that is pharmaceutically orally ingestible. As examples of the polymeric thickener, a substance having the above-described properties, and having a viscosity of 50 mPa•s in 2% aqueous solution to a viscosity of 10000 mPa•s in 0.1% aqueous solution, as determined using a type B viscometer, can be mentioned.

Polymeric thickeners can also be classified as natural polymeric thickeners, semi-synthetic polymeric thickeners, and synthetic polymeric thickeners.

As examples of the semi-synthetic polymeric thickener that can be used in the present invention, water-soluble cellulose derivatives, for example, such as carboxymethylcellulose (hereinafter, also referred to as CMC), CMC sodium salt, CMC potassium salt, CMC calcium salt, methylcellulose, hydroxyethylcellulose, hydroxypropylcellulose, and hydroxypropylmethylcellulose, starch derivatives, for example, such as carboxymethyl starch, starch phosphoric acid ester sodium, and acrylic acid starch, alginic acid derivatives, for example, such as alginic acid polypropylene glycol ester, succinated gelatin and the like can be mentioned.

As examples of natural polymeric thickener that can be used in the present invention, bean-derived polymeric polysaccharides, for example, such as guar gum, carob bean gum, and tamarind seed gum, marine alga-derived polymeric polysaccharides, for example, such as carrageenan and alginic acid Na, microorganism-derived polymeric polysaccharides, for example, such as xanthan gum and gellan gum, fruit-derived polymeric polysaccharides, for example, such as pectin and the like can be mentioned.

As examples of synthetic polymeric thickener that can be used in the present invention, vinyl compounds, for example, such as polyvinyl alcohol, carboxyvinyl polymer, polyvinylpyrrolidone, and polyacrylate sodium and the like can be mentioned.

In the present invention, a single polymeric thickener can be used, but some of the aforementioned thickeners can also be used in combination.

As a more preferable polymeric thickener according to the present invention, as a semi-synthetic polymeric thickener, water-soluble cellulose derivatives can be mentioned. As the most preferable one, carboxymethylcellulose or a salt thereof can be mentioned.

As mentioned herein, the salt of "carboxymethylcellulose salt" refers to, alkali metal salt, for example, such as sodium salt and potassium salt, and alkaline earth metal salt, for example, such as calcium salt and magnesium salt. As a preferable salt, sodium salt can be mentioned.

In constructing the porous dry matrix of the present invention, a polymeric thickener such as carboxymethylcellulose salt, along with excipient ingredient, contributes significantly to the construction of a matrix structure. The role thereof is to support matrix voids in cooperation with the excipient ingredient. More specifically, the polymeric thickener such as carboxymethylcellulose salt is thought to confer strength and shape retaining property to the wall as a structure ingredient.

As the content of the polymeric thickener, such as carboxymethylcellulose salt, in the porous dry matrix preparation (calculated as a content in the composition other than water), from Table 19, one having a content in the range from about 0.5% to about 14% is suitable. Taking eating quality into consideration, more preferably the range from about 0.5% to about 10% can be mentioned. Still more preferably, from Table 23, the range from about 0.5% to about 3.7% can be mentioned.

As mentioned herein, "excipient ingredient" is not particularly limited, as long as it is a substance that functions to support the pores formed at spaces left after evaporation of water, and that is pharmaceutically orally ingestible. As a preferable one, a powder that is almost insoluble in water can be mentioned.

As mentioned herein, "almost insoluble in water" refers to being almost insoluble in water in the ordinary sense; for example, having a solubility of not more than 3 w/w% (the amount of solute that dissolves in 100 g of cold water is not more than 3 g) is referred to as "almost insoluble in water". Particularly, the solubility is preferably not more than 1 w/w%. Referring to the powder that is almost insoluble in water, as examples of an inorganic powder, kaolin (gypsum), diatomaceous earth, talc, hydrated silicon dioxide, light silicic anhydride, magnesium silicate, calcium silicate, calcium phosphate and the like can be mentioned, and as examples of an organic powder, cellulose and cellulose derivatives, for example, such as crystalline cellulose, ethylcellulose, acetic acid-phthalic acid cellulose, and carboxymethylethylcellulose, starches and starch derivatives, for example, such as wheat starch, wheat malt powder, rice starch, maize starch (cornstarch and the like), potato starch, partially pregelatinized starch, dextrin, and hydroxypropyl starch, synthetic polymers, for example, such as polystyrenesulfonate, methacrylate copolymer, ethylene-vinyl acetate copolymer, and crosslinked povidone, furthermore, activated charcoal, magnesium stearate, casein and the like can be mentioned. These excipient ingredients can also be used as blended as appropriate whenever necessary.

Here, polystyrenesulfonate indicates a pharmacologically acceptable polystyrenesulfonate; for example, calcium salt, sodium salt can be mentioned. As a preferable salt, calcium salt can be mentioned. Despite the variability in the particle diameter of the above-described polystyrenesulfonate, in the porous dry matrix preparation of the present invention, a stable impression on ingestion with a constant composition is obtained; moreover, the rough feeling characteristic of polystyrenesulfonate was well masked by the edible oil/fat, and an excellent impression on ingestion was exhibited. Therefore, in the present invention, the particle diameter is preferably as small as possible from the viewpoint of preparation making. As a more preferable one, one having a particle diameter of 5 to 130 $\mu$m can be mentioned. The particle diameter can definitely be determined using a laser diffraction particle size analyzer.

The above-described "excipient ingredient" is used with a polymeric thickener (carboxymethylcellulose salt and the like) to form a structure for a porous dry matrix. Specifically, as the water in a mixed composition comprising excipient ingredient and the like evaporate during thermal drying, pores are produced at spaces left after evaporation of the water. These pores can be dried without undergoing significant volume shrinkage, provided that the content of the wall-forming excipient ingredient and structure ingredient polymeric thickener (e.g., carboxymethylcellulose salt) is sufficient. However, if the content of the excipient ingredient and the polymeric thickener which is a structure ingredient is insufficient, pores cannot be supported, so that the volume of the pores shrinks. This is thought to be roughly the same, for example, as size shrinkage upon drying in paper clay. Therefore, to maintain the shape of the pores at spaces left after evaporation of water, a sufficient amount of an excipient ingredient and a polymeric thickener (e.g., carboxymethylcellulose salt) relative to the water, is necessary. Furthermore, the quantitative ratio of the excipient ingredient and the polymeric thickener (e.g., carboxymethylcellulose salt) is also important from the viewpoint of the strength of the matrix structure.

First, the content of the excipient ingredient in the porous dry matrix preparation (calculated as a content in the composition other than water) is suitably in the range from about 30% to about 80%, taking shape retaining property into consideration, from Table 23. More preferably, the range from about 31% to about 69% is suitable. Likewise, the total content of excipient ingredient and carboxymethylcellulose salt is suitably in the range from about 30% to about 80%, more preferably in the range from about 34% to about 71%.

Regarding ratio of the amounts of the above-described "excipient ingredient" and polymeric thickener (e.g., carboxymethylcellulose salt) added, there is a range suitable for the construction of a porous dry matrix. This ratio of amounts added (relative to the polymeric thickener as 1) is suitably in the range from about 3 to about 100 from Table 18, with shape retaining property as the indicator. More preferably, from Table 23, being in the range from about 10 to about 100 can be mentioned.

The water content in the porous dry matrix preparation of the present invention is preferably not less than 0.1 w/w% and not more than 10 w/w%. A more preferable water content is not less than 0.1 w/w% and not more than 8 w/w%. Here, a measurement of the water content in the porous dry matrix preparation can be performed by, for example, the Karl-Fischer method, or the loss on drying test specified in the Japanese Pharmacopoeia.

As mentioned herein, "water activity" means water necessary for microbial growth and enzyme activity, and is defined as the ratio of the water vapor pressure in the closed container containing a edible (P) to the vapor pressure of pure water at that temperature (P0). Hence, by measuring humidity at the time the inside of the sample-containing closed container reaches an equilibrium (equivalent relative humidity, E.R.H.), water activity (aw) is determined.

If the water activity becomes not more than 0.60 aw, all microorganisms will no longer capable of growing. Therefore, as an example of a preferable water activity of the porous dry matrix preparation of the present invention, a water activity not less than 0.001 and not more than 0.60 can be mentioned.

As mentioned herein, "void ratio" refers to a volume rate of pores contained in a porous dry matrix preparation. Generally, "void ratio" is also referred to as "apparent porosity", which indicates the ratio of the volume of pores or voids to the entire volume in a porous entity consisting of a material and pores or voids, and is calculated using the equation shown below ("Takozairyo" page 32, edited by Renichi Kondo, Gihodo, published in 1973).

$$\text{``Void ratio''} \text{ (porosity)} = \text{(water-saturated weight} - \text{dry}$$
$$\text{weight)} \div \text{(water-saturated weight} - \text{weight in water)}$$
$$\times 100\%$$

In calculating the "void ratio" as mentioned herein, a medium-chain fatty acid triglyceride (specific gravity 0.943) was used in place of water since, when the preparation of the present invention is impregnated with water, some preparations sometimes become difficult to handle because they absorb water and swell or become likely to collapse; the apparent "void ratio" was calculated using the calculation equation shown below.

$$\text{``Void ratio''} \text{ according to the present invention} =$$
$$[(\text{``weight of preparation after immersion in triglyceride under}$$
$$\text{reduced pressure for 5 minutes''} - \text{``weight of dry preparation}$$
$$\text{before immersion''}) \div 0.943]$$
$$\div (\text{``the foregoing weight of preparation after}$$
$$\text{immersion''})$$
$$\times 100\%$$

The sample used had a sample thickness of almost 3 mm; about 1 g of the sample was taken, and the void ratio of the sample was measured.

In the present invention, the void ratio has a positive correlation with water content. Specifically, when water in the mixed composition are evaporated by the thermal drying treatment described in a fourth aspect of the present invention below, the spaces left after evaporation of the water become pores (voids) in the dry matrix while shrinking to some extent. Therefore, the rough upper limit of the void ratio depends on the water content in the mixed composition before drying. For this reason, as the water content increases, the void ratio tends to increase. However, as the water content increases, it becomes more likely that a rough matrix is formed, and a dry matrix with uniform pores tends to be unlikely to be formed. In a dry matrix having a high water content, the matrix puncture, matrix surface bursting and the like occur, and poor appearance and tendency toward matrix collapse and the like occur, so that shape retaining property is often poor. For example, in the case of water content exceeding about 70%, shape retaining property is poor. Meanwhile, as the water content decreases, the void ratio decreased more, and impression on ingestion (tooth touch) tended to worsen. Therefore, the void ratio according to the present invention has a positive correlation with impression on ingestion; it was demonstrated that a dry matrix having a void ratio of not less than about 20% and not more than about 70% offers a good impression on ingestion. A more preferable void ratio is not less than about 25% and not more than about 70%; it was demonstrated that to obtain a porous dry matrix preparation that offers the most preferable impression on ingestion, one having a void ratio of not less than about 30% and not more than about 70% is desirable.

As mentioned herein, "impression on ingestion" indicates a comprehensive sensation combining the three aspects of tooth touch, tongue touch, and flavor. Because the major factor that influences the impression on ingestion is an excipient ingredient, it is possible to improve impression on ingestion by choosing appropriate additives according to the eating quality of the excipient ingredient. For example, for an excipient ingredient with a poor tongue touch, a given amount of edible oil/fat can be added to mask the same. For an excipient ingredient with a poor tooth touch, it is possible to enhance the good impression on ingestion by adding a sugar and/or a sugar alcohol thereto while securing a given void ratio. Furthermore, to improve the flavor, a sweetener, pH regulator, antioxidant and the like can be added according to the intended use as appropriate.

As examples of the sweetener, aspartame, glucose-fructose syrup, reduced maltose syrup, powdered reduced maltose syrup, saccharin, saccharin sodium, stevia, thaumatin, erythritol, sorbitol, sorbitol liquid, mannitol, glucose, lactose, white sugar, fructose, honey, xylitol, glycerin, concentrated glycerin, propylene glycol, maltitol, maltitol liquid, trehalose and the like can be mentioned, and the sweetener may be a mixture thereof. As preferable sweeteners, white sugar, powdered reduced maltose syrup, sorbitol, mannitol, glucose, and saccharin sodium can be mentioned.

Furthermore, to impart a smell for improving the flavor, a flavor can also be added. As the flavor, generally used ones such as vanilla essence and coffee flavor can be used.

As examples of the pH regulator, a buffer agent consisting of an organic acid such as citric acid, tartaric acid, lactic acid, fumaric acid, or malic acid and an alkali metal salt thereof, and a buffer agent of, for example, an inorganic acid such as phosphoric acid and an alkali metal salt thereof can be mentioned. The porous dry matrix preparation of the present invention, as described in the fourth aspect of the present invention below, is normally produced by mixing at least a polymeric thickener, an excipient ingredient and water to give a composition, molding same and thermally drying the molded product, and the liquid nature of the mixed composition tends to desirably have an acidic pH. For example, a porous dry matrix preparation from a mixed composition whose liquid nature is on the acidic side tends to be obtained as a hydrated product with higher strength (hydrated matrix obtained by impregnating a dry matrix with water). As the antioxidant, ascorbic acid, sodium hydrogen sulfite, sodium sulfite, erythorbic acid, tocopheryl acetate, dibutylhydroxytoluene, tocopherol, sodium pyrrosulfite, butylhydroxyanisole, propyl gallate and the like can be mentioned.

As mentioned herein, "sugar" includes white sugar, lactose, glucose, liquid sugar, fructose, glucose-fructose syrup, caramel, powdered reduced maltose syrup, brown sugar, simple syrup, powdered sugar, starch syrup, maltose, powdered starch syrup, honey, high-glucose syrup and the like, and is not particularly limited. Any choice of sugar can be used.

The "sugar alcohol" according to the present invention includes, but is not particularly limited to, sorbitol, mannitol, xylitol, glycerin, erythritol, maltitol and the like; any kind of sugar alcohol can be used.

In the present invention, sugars and sugar alcohols are not only used as sweeteners to improve the flavor, but also thought to be effective in increasing the extension of a mixed composition during the kneading of the mixed composition described in a fourth aspect of the present invention below. Therefore, for a mixed composition wherein the excipient ingredient is likely to be dispersed, kneading can be effectively performed by adding these sugars or sugar alcohols. It was found that regarding the amount of these sugars or sugar alcohols added, they can be added over the wide range from about 8% to about 42% as contents in the porous dry matrix preparation in terms of shape retaining property (according to Table 7), and that the influence on void ratio is small.

Meanwhile, it was found that for a preparation wherein the excipient ingredient has a good eating quality and is likely to gather, like wheat flour, a desired porous dry matrix preparation can be prepared without adding a sugar or a sugar alcohol as in sample No.61.

As mentioned herein, "an edible oil/fat" refers to a fatty acid triglyceride-series substance that is liquid or solid at normal temperature or a mixture thereof; specifically, liquid edible oil (fat oil) or glycerin and a fat that is solid at normal temperature can be mentioned. The edible oil/fat the porous dry matrix preparation of the present invention can comprise is not particularly limited, as long as it can be used to mask the eating quality of excipient ingredient; salad oil, sesame oil, cottonseed oil, safflower oil, coconut oil, medium-chain fatty acid triglycerides, peanut oil, almond oil, corn oil, olive oil, canola oil, soy oil, rapeseed oil, palm oil, oleic acid, sunflower oil, wheat germ oil and the like can be mentioned. As the solid fat according to the present invention, butter, tallow, stearic acid and the like can be mentioned. As the edible oil/fat according to the present invention, an edible oil is preferable; for example, medium-chain fatty acid triglycerides, salad oil, sesame oil, cottonseed oil, safflower oil, corn oil, olive oil, canola oil, soy oil, rapeseed oil, palm oil, peanut oil can be mentioned. As the most preferable ones, medium-chain fatty acid triglycerides and safflower oil can be mentioned. As examples of glycerin, glycerin solutions and concentrated glycerin can be mentioned. These can be used singly or in combination according to the intended use.

It was also found that as the roles of the edible oil/fat, not only the eating quality of excipient ingredient is masked, but also the edible oil/fat being dispersed in the mixed composition facilitates heat conduction to the inside of the mixed composition during thermal drying, and as a result, the evaporation of the water inside of the mixed composition can be accelerated, to prepare a uniform matrix with communicated pores. It was found that the thus-obtained uniform porous dry matrix preparation with communicated pores has a good water absorption capacity at the time of re-impregnation with water, and tends to have excellent shape retaining property after water absorption.

The amount of edible oil/fat added changes mainly depending on the eating quality of excipient ingredient, and it was found that the void ratio decreases according to the amount added. Therefore, as the amount of edible oil/fat added, an amount such that the void ratio would not become not more than about 20% can be added. For example, from Table 11, it was found that in terms of impression on ingestion, the edible oil/added can be used in the range exceeding about 33% as the content in the porous dry matrix preparation.

As the amount of edible oil/fat increases, the inside of the porous dry matrix preparation tends to be increasingly filled, so that the void ratio decreases. For this reason, it is desirable that the amount of edible oil/fat added be adjusted so that the void ratio would be not less than 25%, more preferably not less than 30%.

It was found that as the amount of edible oil/fat added to achieve a desired void ratio, to impart a flavor, and to cover the surface of the matrix structure to improve impression on ingestion, since the amount depends on the amount of excipient ingredient, as the numerical value for edible oil/fat/(excipient ingredient + CMCNa and the like), around about 0.5 is sufficient. Taking the void ratio into consideration, it is thought that the above-described ratio of amounts added is desirably not more than about 0.7. More preferably, amounts added of not more than about 0.6 can be mentioned.

Furthermore, when polystyrenesulfonate is used as the excipient ingredient, to ameliorate the flouriness characteristic of polystyrenesulfonate using a edible oil/fat, it is desirable that preferably at least not less than 0.06 parts by weight, more preferably not less than 0.12 parts by weight, most preferably not less than 0.13 parts by weight, per part by weight of polystyrenesulfonate, of a edible oil/fat be contained.

In "Yakuri-To-Chiryo (Japanese Pharmacology & Therapeutics), Vol.21, No.6. 379 (1993)", to ameliorate this bad impression on ingestion of polystyrenesulfonate, attempts were made to mask the discomfort of polystyrenesulfonate by blending it with various foods. For example, eight items, including madeleines, steamed bread, fried balls, and cookies, were investigated. However, it has been shown that it is difficult to achieve an improvement in impression on ingestion unless the content of the polystyrenesulfonate is not more than about 7 to about 9%, one wherein the content of the polystyrenesulfonate exceeds 60%, like in the present invention, is beyond the scope of the assumption. Additionally, the fact that the discomfort of polystyrenesulfonate is masked by a small amount of not less than 0.12 parts by weight of edible oil/fat, as described above is also a feature of the porous dry matrix preparation of the present invention.

Furthermore, to facilitate the blending of an edible oil/fat and water and the like to more uniformly disperse the edible oil/fat, an emulsifier (surfactant) can be added. Addition of these surfactants tended to produce smaller pore sizes, facilitating the communication of pores to the inside. For this reason, it is possible to further improve the impression on ingestion.

The choice of emulsifier added is not particularly limited, as long as it is pharmaceutically orally ingestible; for example, an emulsifier for foods, polyvinyl alcohol, ethylene glycol, propylene glycol, polysorbates and the like can be used. Examples of the emulsifier for foods include glycerin fatty acid esters, for example, such as acetic acid monoglyceride, lactic acid monoglyceride, citric acid monoglyceride, diacetyltartaric acid monoglyceride, and succinic acid monoglyceride, saponins, for example, such as quillaia extract, soybean saponin, and tea seed saponin, sucrose fatty acid esters obtained, for example, by reaction of acetic acid, isobutyric acid, stearic acid, palmitic acid, oleic acid or the like with sucrose, and lecithins, for example, such as vegetable lecithins from soybean and oilseed rape, and egg-yolk lecithin. Examples of sorbates include Tween 80, monooleic acid polyoxyethylenesorbitan and the like can be mentioned.

- Second aspect of the present invention -

A second aspect of the present invention relates to a porous dry matrix preparation containing a desired pharmacologically effective ingredient. Accordingly, this aspect corresponds to a case wherein the porous dry matrix preparation of the first aspect described above contains a pharmacologically effective ingredient.

As mentioned herein, "a pharmacologically effective ingredient" refers to an orally administrable drug, and is not particularly limited, as long as it is orally ingestible. Hereinafter, the term "drug" is used to have the same meaning as "pharmacologically effective ingredient".

In the case of a stable drug that endures thermal drying at 140°C or higher, as described in the fourth and fifth aspects of the present invention below, the drug can be simultaneously kneaded in the mixed composition before thermal drying, and the kneaded mixture can be subjected to thermal drying treatment as is to obtain a desired porous dry matrix preparation. As examples of the drug, amino acids such as valine, leucine, and isoleucine, theophylline, salazosulfapyridine, indomethacin, diclofenac sodium, loxoprofen, metformin hydrochloride and the like can be mentioned. Furthermore, a drug is sometimes used as an excipient ingredient; such drugs include calcium polystyrenesulfonate, sodium polystyrenesulfonate, activated charcoal, salazosulfapyridine, Chinese herbal medicine, crude drugs and the like.

Meanwhile, a drug that is unstable to heating and the like, as described in the fourth and fifth aspects of the present invention below, can be dissolved in a solvent such as water and oil, and impregnated to a porous dry matrix preparation, and the solvent is distilled off and the like, whereby a porous dry matrix preparation containing the pharmacologically effective ingredient can be prepared. Because ordinary drugs are not highly stable to heat, it is desirable that they be dissolved in a solvent, and impregnated in the porous dry matrix preparation. The solvent used in this method is not particularly limited, as long as it is orally usable; for example, purified water, aqueous solutions such as pH-adjusted buffer solutions, alcohol-series solvents, for example, such as ethanol, ethylene glycol, isopropanol, isobutanol, propylene glycol, 1,3-butanediol, and glycerin, edible oils mentioned in the above-described "edible oil/fat", for example, such as medium-chain fatty acid triglycerides and corn oil, ether-series solvents, for example, such as ethyl ether and 1,2-dimethoxyethylene, acetic acid ester-series solvents, for example, such as ethyl acetate and the like can be mentioned. Furthermore, after drug is impregnated, to improve impression on ingestion, a solution of a sweetener and the like is sprayed over the surface of the porous dry matrix preparation, or is further impregnated, whereby a further improvement in the impression on ingestion can be achieved. The drug that can be used in the above-described method is not particularly limited, and as preferable ones, drugs in use for children can be mentioned. Anti-allergic agents, for example, such as Zyrtec, Ebastel, and ketotifen, cold remedies, antipyretic analgesic agents, for example, such as ketoprofen, ibuprofen, Loxonin, and acetaminophen, antidiarrheal drugs such as loperamide hydrochloride, bronchodilators such as tulobuterol hydrochloride and procaterol hydrochloride, expectorant drugs such as carbocysteine and ambroxol hydrochloride, antitussive drugs, anthelmintics and the like can be mentioned as suitable drugs.

The other terms as used herein are as defined in the aspects described above.

- Third aspect of the present invention -

A third aspect of the present invention relates to a dry matrix preparation that has particularly good water absorption capacity and absorbs water to become a porous hydrated matrix with a good impression on ingestion (hydrated composition) out of the porous dry matrix preparation in the first aspect or the second aspect described above.

As mentioned herein, "a hydrated composition" refers to a porous dry matrix preparation that has become jelly-like, finely textured, and sponge-like as a result of absorption of water.

As mentioned herein, "water" means water or one of various beverages. A beverage used can be chosen according to the intended use as appropriate; a porous matrix preparation impregnated with water offers a still better flavor and impression on ingestion. As common beverages, common drinks such as coffee, black tea, green tea, oolong tea, juices, and sport drinks can be widely used.

Provided that the excipient ingredient and the drug are both calcium polystyrenesulfonate, taking ion exchange with potassium, which is the intended use, into consideration, a beverage refers to water, or various low-potassium beverages, for example, such as black tea, green tea, barley water, oolong tea, low-potassium processed juices, carbonated drinks, colas. Because a broad range of common beverages can be used, a characteristic flavor based thereon can be imparted to wet preparation (hydrated composition) by the beverage to be impregnated.

As mentioned herein, "polymeric thickener" and "carboxymethylcellulose salt" are as defined above; regarding the content thereof, an appropriate content (calculated as a content in the composition other than water) is determined, with shape retaining property after re-impregnation of the preparation with the water being the indicator; from Table 19, the range from about 0.5 to about 14% is thought to be suitable as the content in the dry matrix preparation. Taking Table 23 into consideration, the range from about 0.5 to about 3.7 can be mentioned as a more preferable range. For producing a porous dry matrix preparation in this aspect, it is normally preferable to use a highly viscous polymeric thickener; for example, one having a viscosity of not less than 1000 mPa•s in 1% aqueous solution, more preferably a viscosity of not less than 3000 mPa•s in 1% aqueous solution, as determined using a type B viscometer, can be mentioned.

Also, regarding to the quantitative ratio of the polymeric thickener (e.g., carboxymethylcellulose salt) and the excipient ingredient (excipient ingredient/CMCNa and the like), appropriate quantitative ratio (relative to the polymeric thickener as 1) is determined with shape retaining property after re-impregnation with water being the indicator; from Table 17, it is desirable that the ratio be in the range from about 5 to about 100. Taking the pore volume maintenance rate into consideration, from Table 18, it is desirable that the ratio be in the range from about 10 to about 100.

The above-described "shape retaining property" refers to a state wherein in the preparation obtained, the molded shape is maintained, and there are no major cracks or major pore ruptures, and the preparation does not get out of shape when held in a hand. The same applies to shape retaining property after water absorption, referring to a state wherein the preparation does not get out of shape when held in a hand. Shape retaining property refers to, for example, strength such that the preparation can be pinched up with fingers after being re-impregnated with water.

Since the influence of the excipient ingredient on eating quality increases as the content of the excipient ingredient is increased, a sweetener and edible oil/fat may be added as appropriate so as to improve the impression on ingestion and water absorption capacity of the porous dry matrix preparation obtained. A plurality of sweeteners and edible oils/fats may be used in combination. Furthermore, to increase the water absorption capacity, a pH regulator and the like may be added.

As mentioned herein, "water absorption capacity" is evaluated by the time taken for the water to permeate the inside of the porous dry matrix preparation. Specifically, starting at the time when a intended porous dry matrix preparation is immersed in the water, time taken for the touch of the impregnated sample being pinched with fingers to become a state wherein no core is observed in the sample is measured; if the water absorption time thus determined is within 30 minutes, the sample is judged to have passed the test. A more preferable one refers to one having a water absorption time of within 15 minutes after immersion. A still more preferable one refers to one having a water absorption time of within 5 minutes after immersion.

Water absorption time tends to be influenced by sample thickness and size. In the present invention, a sample having an almost constant sample thickness of about 3 mm or about 4 mm is prepared, and the test was performed using samples of somewhat constant size.

Describing from the viewpoint of physical properties, as uniformly dispersed pores are communicated to the inside of the porous dry matrix preparation, water absorption time tends to shorten. Therefore, a porous dry matrix preparation having a somewhat short water absorption time is one wherein pores are finely uniformly dispersed to the inside of the porous dry matrix preparation, and communicate with each other. Such a porous dry matrix preparation becomes a preparation with an elastic jelly-like touch that offers a good impression on ingestion when re-impregnated with water. Hence, water absorption time tends to be correlated with void ratio; water absorption time tends to shorten as the void ratio increases. Therefore, a porous dry matrix preparation that offers a water absorption time of within 5 minutes is preferable in terms of void ratio and impression on ingestion. A more preferable porous dry matrix preparation is one having a water absorption time of within 1 minute. Considering in terms of void ratio, to allow water to permeate the inside of the porous dry matrix preparation within 1 minute, it is desirable that the void ratio be not less than 29%.

The other terms as used herein are as defined in the aspects described above.

- Fourth aspect of the present invention -

A fourth aspect of the present invention relates to a method of producing a porous dry matrix preparation.

As used in this aspect, the terms "polymeric thickener", "carboxymethylcellulose salt", and "excipient ingredient" and the like are as defined in the aspects described above.

In this aspect, "porous dry matrix preparation" is as described in the first aspect described above. Specifically, the porous dry matrix preparation produced by the method of production in this aspect is specifically exemplified by the porous dry matrix preparations in the first to third aspects described above.

The method of production in this aspect comprises subjecting to thermal drying a mixed composition obtained by mixing and molding at least a polymeric thickener, an excipient ingredient, and water.

Regarding the total content of the polymeric thickener (e.g., carboxymethylcellulose salt) and the excipient ingredient in the mixed composition (calculated as a content in the composition other than water) before drying, with shape retaining property as the index; from Table 23, it is suitable that the total content be in the range from about 15% to about 60%.

More preferably, judging from Table 21, the total content ranges from about 20% to about 45%.

The content of each of the polymeric thickener (e.g., carboxymethylcellulose salt) and the excipient ingredient before drying (calculated as a content in the composition other than water) can be the content of each of the polymeric thickener and excipient ingredient in the porous dry matrix preparation to be produced, being within the above-described range of suitable total contents of polymeric thickener and excipient ingredient before drying.

As mentioned herein, "water content before drying" refers to the calculated water content in the mixed composition before drying. Taking the fact that the upper limit of the void ratio depends nearly essentially on the water content in the mixed composition before drying, as described above, into consideration, from the results in Table 13, to meet the numerical requirement for a void ratio of not less than about 20%, the water content in the mixed composition need to be not less than about 25%.

As mentioned herein, "to mold" refers to casting a mixed composition kneaded in a clay form into a given frame, or preparing the composition as a dough with a given thickness and cutting the composition into a given shape. Because drying method, drying time, and water absorption capacity after drying are influenced by this molding size, appropriate thickness and length-width dimensions of molding size can be determined in relation to production efficiency. In the present invention, because the molding conditions were set so that drying would be completed within 30 minutes, for example, a thickness of about 3 to 4 mm and a length-width size of about 6 to 7 cm x about 2 to 3 cm are suitable.

Regarding the influence of drying on initial molding size, initial molding size tends to be maintained in terms of thickness, but length-width size tends to be somewhat variable according to water content.

As mentioned herein, "thermal drying" is not particularly limited, as long as it represents a means for evaporating water; however, to achieve efficient production of a dry matrix preparation, it is desirable that thermal drying be performed under normal pressure. Drying temperature is particularly important; at low drying temperatures, it tends to be unlikely that a matrix having an appropriate void is produced. To appropriately evaporate the water contained in the inside of the mixed composition, and to prepare a void wherein pores communicate with each other in a good shape to the inside, a temperature of 100˚C or higher is necessary. Preferably, a heating temperature of about 120˚C or higher is necessary, furthermore, it is desirable that the thermal drying be performed at a temperature under the level at which thermal decomposition and thermal denaturation of ingredients in the composition are not caused.

Therefore, the heating temperature is suitably in the range from about 100˚C to about 200˚C, more preferably about 120˚C to 170˚C, and thermal drying is suitably performed still more preferably at a temperature of around about 150˚C. Furthermore, in the case of a mixed composition with a high water content, plural times of the thermal drying treatment can be performed; for example, thermal drying is performed at a relatively low temperature of 60˚C to 130˚C for a short time to evaporate water to some extent, and thereafter thermal drying is performed at a temperature of 140˚C to 150˚C or higher and the like. In the case of excipient ingredients and other additives that are likely to release water, drying is possible at lower temperatures; for example, heating at a temperature of 120˚C or higher is possible.

Regarding the means for heating, ordinary means of heating can be used; for example, hot air drying, hot plate drying and the like can be mentioned. Regarding the orientation of heating, both one-face drying and both-face drying are applicable. In heating with a hot plate as well, both both-face heating and one-face heating are available; an appropriate method can be chosen according to the water content and size of the mixed composition, or drying time and other conditions. Furthermore, taking the water content and size of a mixed composition, or drying time and the like into consideration, it is also possible to obtain a desired porous dry matrix preparation by pre-heating a mixed composition around 100˚C, for example, at about 60˚C to about 120˚C, preferably about 80˚C to about 120˚C, and thereafter performing a thermal drying treatment according to the present invention.

Heating time means a time taken to reach the drying endpoint. The drying endpoint is suitably determined as the time when the water activity measured has become not more than 0.60, or the time when the water content has become not more than 10 w/w%. As the endpoint, more preferably, a time when the water activity content has become not more than 0.55, and the water content has become not more than 8% can be mentioned.

- Fifth aspect of the present invention -

A fifth aspect of the present invention relates to a method of producing a porous dry matrix preparation comprising a desired pharmacologically effective ingredient by means of a porous dry matrix. A porous dry matrix preparation produced by the method of production in this aspect is, for example, the porous dry matrix preparation in the second aspect described above.

As shown in the second aspect of the present invention, the pharmacologically effective ingredient may be added from before drying, or may be incorporated in the porous dry matrix preparation by impregnation after drying and the like. The choice of the method depends on the stability of the pharmacologically effective ingredient. In the case of a pharmacologically effective ingredient that is thermally stable even at the thermal drying temperature for evaporating the water, preparation making may be performed in the same manner as the fourth aspect described above; that is, along with other ingredients, before drying, the pharmacologically effective ingredient can be kneaded in a mixed composition, molded, and thermally dried to yield a desired porous dry matrix preparation. Meanwhile, in the case of a pharmacologically effective ingredient that is thermally unstable at the heating temperature, a desired porous dry matrix preparation can be obtained by using a porous dry matrix composition separately obtained by the method described in the fourth aspect described above, impregnating this with a solution of a pharmacologically effective ingredient, and evaporating the solvent for the solution so that the water activity or water content will become under the specified levels.

As the solution for the pharmacologically effective ingredient, if the pharmacologically effective ingredient is freely soluble in water, the solution is prepared using purified water. Meanwhile, if the pharmacologically effective ingredient is almost insoluble in water, the same is dissolved in a highly evaporative organic solvent or a pharmaceutically orally ingestible oily medium to yield a solution. Examples of the organic solvent used include alcohol-series solvents, for example, such as ethyl alcohol, ether-series solvents, for example, such as ethyl ether and 1,2-dimethoxyethylene, ester-series solvents such as ethyl acetate and the like; any solvent can be used, as long as it is readily evaporable, and is not pharmaceutically problematic even if remaining in trace amounts. These solvents can also be used in combination as appropriate. As examples of the oily medium used, the above-described "edible oil/fat" and the like can be mentioned.

A pharmacologically effective ingredient concentration in the solution is chosen according to the intended use as appropriate. As a method of impregnating the solution of the pharmacologically effective ingredient into a porous dry matrix composition, a common method can be chosen according to the intended use as appropriate. For example, a given amount of the solution can be injected into the inside or surface of the porous dry matrix composition using a syringe to impregnate the composition. Furthermore, a given amount can be coated by means of a brush, spray and the like.

If the eating quality is not good due to bitterness of the pharmacologically effective ingredient and the like, it is recommended that improvements such as masking the bitterness be performed while the ingredient is in solution, and the solution be injected, coated or impregnated.

After solution impregnation and the like, to evaporate the water of the solution, organic solvent or oil that are present in the porous dry matrix composition, drying at a low temperature, drying under reduced pressure and the like can be performed in combination as appropriate.

The drying endpoint is suitably determined as the time when the water activity measured has become not more than 0.60, or the time when the water content has become not more than 10 w/w%. As the endpoint, more preferably, a time when the water activity content has become not more than 0.55, and the water content has become not more than 8% can be mentioned.

The other terms as used herein are as defined in the aspects described above.

Examples

The present invention is hereinafter described in more detail by means of the following Examples, but the scope of the present invention is never limited to the Examples below. All amounts blended in the Examples below are expressed as gram mass.

Example 1: Investigation of thermal drying treatment

Thermal drying treatment as a substitute for conventional freeze-drying treatment was investigated as follows. First, 1.0 g of carboxymethylcellulose sodium, 5 g of crystalline cellulose, 5 g of a medium-chain fatty acid triglyceride, 4 g of white sugar and 15 g of purified water were mixed and kneaded to yield a clay-like composition. This was elongated, and molded to have a sample thickness of about 3 mm and a length-width size of about 6.6 cm x about 2.6 cm. Using this sample, thermal drying was performed at each of the temperatures shown below.

When the preparation water activity became not more than 0.55, the sample was allowed to cool, and the porous dry matrix preparation was preserved in a sealed container. The porous dry matrix preparation obtained maintained a thickness of about 3 mm. To perform a dosage form evaluation of this preparation, the shape retaining property and void ratio of the preparation were determined. The results are shown in Table 1 below.

[Table 1]

| Test No. | Thermal drying temperature | Void ratio | Dry preparation | | | After water absorption | |
|---|---|---|---|---|---|---|---|
| | | | Shape retaining property | Impression on ingestion | Water absorption capacity | Shape retaining property | Impression on ingestion |
| 1 | 80˚C/one face | 16.5% | ◯ | ✕ | ✕ | ✕ | ✕ |
| 2 | 100˚C/one face | 17.4% | ◯ | ✕ | ✕ | ✕ | ✕ |
| 3 | 120˚C/one face | 19.0% | ◯ | ◯ | ✕ | ✕ | ✕ |
| 4 | 120˚C/both faces | 19.7% | ◯ | ◯ | ✕ | ✕ | ✕ |
| 5 | 140˚C/both faces | 25.9% | ◯ | ◯ | ◯ | ◯ | ◯ |
| 6 | 150˚C/both faces | 26.7% | ◯ | ◯ | ◯ | ◯ | ◯ |
| 7 | 170˚C/both faces | 29.4% | ◯ | ◯ | ◯ | ◯ | ◯ |
| 8 | 200˚C/both faces | 27.4% | ◯ | ✕ | ◯ | ◯ | ◯ |
| [Note] ◯: good, ✕: poor, △: equivocal. | | | | | | | |

Regarding water absorption capacity, a sample with water absorption time within 15 minutes was given the rating "good", and a sample with water absorption time of not less than 30 minutes was given the rating "poor".

From this result, it was found that by thermal drying at a temperature of 120˚C or higher, the water in the mixed composition evaporates effectively, and a puffed-up porous dry matrix preparation having pores communicating with each other to the inside of the composition is obtained. It was also found that the impression on ingestion of the porous dry matrix preparation correlates with void ratio, and is significantly influenced by void ratio. As shown by this result, as the void ratio decreases below about 20%, the impression on ingestion (tooth touch) tends to worsen. With drying at 200˚C, white sugar scorched, and the overall rating of impression on ingestion was poor. Furthermore, when the shape retaining property and impression on ingestion of a porous hydrated matrix preparation prepared by impregnating a porous dry matrix preparation with water was investigated, it was demonstrated that shape retaining property and impression on ingestion are poor unless the void ratio is not less than about 20%.

In the following investigation of the porous dry matrix preparation, thermal drying temperature was set at about 150˚C, and pharmaceutical formulations were investigated.

Example 2: Amount of composition added and void ratio

To identify the essential factors for the composition of Example 1, the composition amounts (numbers of grams) listed in Table 2 below were weighed out. 1.0 g of carboxymethylcellulose sodium, 5 g of crystalline cellulose, 5 g of a medium-chain fatty acid triglyceride, the sugar or sugar alcohol shown in Table 2 below (4 g) and 15 g of purified water were mixed at room temperature, and thermally dried at about 150˚C to yield a porous dry matrix preparation.
A preparation was produced in the same manner as Example 1, and when the water activity of the preparation became not more than 0.55, or when the amount of water contained in the preparation became not more than 10 w/w%, heat treatment was finished. The void ratio and shape retaining property of the porous dry matrix preparations obtained are shown in Table 2 below. As shown by this result, it was found that as the amount of substances added increased, the void ratio tended to decrease. It was also found that void ratio correlates with the amount of water contained before drying.
[Table 2]

|  | No.1 | No.2 | No.3 | No.4 | No.5 | No.6 |
|---|---|---|---|---|---|---|
| Carboxymethyl cellulose sodium | 1.0 | 1.0 | 1.0 | 1.0 | 1.0 | 1.0 |
| Crystalline cellulose | 5.0 | 5.0 | 5.0 | 5.0 | 5.0 | 5.0 |
| Purified water | 15.0 | 15.0 | 15.0 | 15.0 | 15.0 | 15.0 |
| White sugar |  | 4.0 | 4.0 | 4.0 | 4.0 | 4.0 |
| Medium-chain fatty acid triglyceride |  |  | 1.0 | 5.0 | 5.0 | 5.0 |
| Citric acid |  |  |  |  | 0.01 |  |
| Sodium citrate |  |  |  |  | 0.1 |  |
| Sodium malate |  |  |  |  |  | 0.2 |
| Amount of water contained before drying | 71.4% | 60.0% | 57.7% | 50.0% | 49.8% | 49.7% |
| void ratio | 50.1% | 42.1% | 35.4% | 27.8% | 27.1% | 25.3% |
| Shape retaining property | ○ | ○ | ○ | ○ | ○ | ○ |
| Impression on ingestion | △Hard and floury | ○ | ○ | ○ | ○ | ○ |
| Water absorption capacity | × | ○ | △ | ○ | ○ | ○ |
| After water absorption: Shape retaining property | ○ | ○ | ○ | ○ | ○ | ○ |
| Impression on ingestion | ○ | ○ | ○ | ○ | ○ | ○ |
| [Note] ○: good, ×: poor, △: equivocal. | | | | | | |

This void ratio reduction shows that carboxymethylcellulose sodium (hereinafter, also abbreviated "CMCNa"), which is a polymeric thickener, and crystalline cellulose, which is an excipient ingredient, form pores of a porous dry matrix, and that the pores (pore volume) tend to shrink with drying, and to be filled by other ingredients. Hence, using pore volume maintenance rate during thermal drying (void ratio/amount of water contained), an evaluation of sample No. 1 to No.6 was performed. The results are shown in Table 3 below.
[Table 3]

|  | No.1 | No.2 | No.3 | No.4 | No.5 | No.6 |
|---|---|---|---|---|---|---|
| Amount of water contained before drying | 71.4% | 60.0% | 57.7% | 50.0% | 49.8% | 49.7% |
| Pore volume maintenance rate | 70.2% | 70.2% | 61.4% | 55.6% | 54.4% | 50.9% |
| Shape retaining property | × | ○ | ○ | ○ | ○ | ○ |
| Impression on ingestion | △Hard and floury | ○ | ○ | ○ | ○ | ○ |

According to this result, as the amount of water contained decreased, void ratio and pore volume maintenance rate decreased. As ingredients other than CMCNa and excipient ingredient (crystalline cellulose) (white sugar and medium-chain fatty acid triglyceride) increased, the pore volume maintenance rate decreased. However, sample No.1 to No.6 after drying had nearly the same apparent external size capacity. Therefore, the reduction in pore volume maintenance rate despite the constant external size of each sample is attributable to a void ratio reduction due to the accumulation of ingredients in excess for matrix construction in the matrix pores produced after drying (pores left at sites after evaporation of water).

Regarding impression on ingestion and flavor, in sample No.1 (void ratio 50.1%), both tooth touch and tongue touch were poor, and the flouriness of crystalline cellulose was conspicuous. Hence, it was found that by adding white sugar or a medium-chain fatty acid triglyceride, the flouriness of crystalline cellulose can be successfully masked to significantly improve the tongue touch.

Example 3: Effects of addition of composition that influence the void ratio

To determine the effects of addition of a sugar and medium-chain fatty acid triglyceride out of the ingredients shown in Example 2, a porous dry matrix preparation not containing sugar (No.7 in Table 4 below) was newly prepared. The preparation was prepared in the same manner as Example 2; the void ratio and shape retaining property of the porous dry matrix preparation obtained are shown in Table 4 below.

[Table 4]

|  | No.1 | No.2 | No.7 | No.4 |
|---|---|---|---|---|
| Carboxymethyl-cellulose sodium | 1.0 | 1.0 | 1.0 | 1.0 |
| Crystalline cellulose | 5.0 | 5.0 | 5.0 | 5.0 |
| Purified water | 15.0 | 15.0 | 15.0 | 15.0 |
| White sugar |  | 4.0 |  | 4.0 |
| Medium-chain fatty acid triglyceride |  |  | 5.0 | 5.0 |
| Amount of water contained before drying | 71.4% | 60.0% | 57.7% | 50.0% |
| Preparation void ratio | 50.1% | 42.1% | 32.0% | 27.8% |
| Shape retaining property | × | ○ | ○ | ○ |
| Impression on ingestion | △Hard and floury | ○ | △Damp and floury | ○ |
| Water absorption capacity | × | ○ | ○ | ○ |
| After water absorption: Shape retaining property | ○ | ○ | ○ | ○ |
| Impression on ingestion | ○ | ○ | ○ | ○ |
| [Note]<br>○: good, ×: poor, △: equivocal. | | | | |

From this result, it was shown that white sugar as the sweetener significantly improves the impression on ingestion. It was also demonstrated that sugar also contributes significantly to the improvement of tooth touch. Meanwhile, it was demonstrated that when used as the edible oil/fat, a medium-chain fatty acid triglyceride masks the impression on ingestion (floury tongue touch) of excipient ingredient (crystalline cellulose) to moisten the preparation and contribute to an improvement in the impression on ingestion.

When an evaluation was performed using pore volume maintenance rate, the results shown in Table 5 below were obtained.

[Table 5]

|  | No.1 | No.2 | No.7 | No.4 |
|---|---|---|---|---|
| Presence or absence of sugar |  | Present |  | Present |

(continued)

| | No.1 | No.2 | No.7 | No.4 |
|---|---|---|---|---|
| Presence or absence of medium-chain fatty acid triglyceride | | | Present | Present |
| Pore volume maintenance rate | 70.2% | 70.2% | 55.5% | 55.6% |
| Shape retaining property | × | ○ | ○ | ○ |
| Impression on ingestion | △Hard and floury | ○ | △Damp and floury | ○ |

As shown by this result, even when sugar was added, the pore volume maintenance rate did not change. From this, it can be concluded that sugar does not act as an excipient ingredient, and is an ingredient that does not contribute to the maintenance of the structure of the porous dry matrix. Meanwhile, when a medium-chain fatty acid triglyceride is added, the eating quality (tongue touch) of porous dry matrix preparation can be improved; however, as shown in Table 5, when the same is added, pore volume maintenance rate decreases. From these findings, it is thought that the triglyceride covers the matrix surface to improve impression on ingestion (tongue touch), but the amount covering the matrix fills the pores, and as a result, the void ratio decreases.
From these findings, it was found that to improve the impression on ingestion of the excipient ingredient, a given amount of sugar is effective in playing a role as a sweetener, and that a given amount of a medium-chain fatty acid triglyceride is effective in improving the impression on ingestion of excipient ingredient (improvement of tongue touch).

Example 4: Effects of the amount of sugar added as sweetener

To investigate the effects of the amount of sugar added, the composition amounts (numbers of grams) listed in Table 6 below were weighed out, and porous dry matrix preparations were prepared in the same manner as Example 2. The void ratio and shape retaining property of the porous dry matrix preparation obtained are shown in Table 6 below.
[Table 6]

| | No.7 | No.8 | No.9 | No.4 | No.10 |
|---|---|---|---|---|---|
| Carboxymethyl-cellulose sodium | 1.0 | 1.0 | 1.0 | 1.0 | 1.0 |
| Crystalline cellulose | 5.0 | 5.0 | 5.0 | 5.0 | 5.0 |
| Purified water | 15.0 | 15.0 | 15.0 | 15.0 | 15.0 |
| White sugar | | 1.0 | 2.0 | 4.0 | 8.0 |
| Medium-chain fatty acid triglyceride | 5.0 | 5.0 | 5.0 | 5.0 | 5.0 |
| Amount of water contained before drying | 57.7% | 55.6% | 53.6% | 50.0% | 44.1% |
| Void ratio | 32.0% | 30.9% | 28.7% | 27.8% | 22.3% |
| Shape retaining property | × | ○ | ○ | ○ | ○ |
| Sugar content after drying | 0% | 8.3% | 15.4% | 26.79% | 42.1% |
| Impression on ingestion | △ | △ | ○ | ○ | ○ |
| Water absorption capacity | ○ | ○ | ○ | ○ | × |
| After water absorption: Shape retaining property | ○ | ○ | ○ | ○ | Not evaluable due to poor water absorption |
| Impression on ingestion | ○ | ○ | ○ | ○ | (Same as above) |
| [Note] ○: good, ×: poor, △: equivocal. | | | | | |

From this result, it was shown that to improve the impression on ingestion of excipient ingredient, in the case of crystalline cellulose, the sugar content in the composition other than water (porous dry matrix) is preferably not less than about 9%.

When this result is expressed by pore volume maintenance rate, the sugar content in the preparation, and the ratio of the excipient ingredient to the sugar, the results shown in Table 7 below are obtained.

[Table 7]

|  | No.7 | No.8 | No.9 | No.4 | No.10 |
|---|---|---|---|---|---|
| Pore volume maintenance rate | 55.5% | 55.6% | 53.5% | 55.6% | 50.6% |
| Sugar content in porous dry matrix preparation | 0% | 8.3% | 15.4% | 26.7% | 42.1% |
| Shape retaining property | × | ○ | ○ | ○ | ○ |
| Impression on ingestion | △ | ○ | ○ | ○ | ○ |

As shown by this result, it was found that until the sugar content became nearly about 40%, the pore volume maintenance rate was constant at a level of about 55%, showing no major change. From this, it is seen that although sugar does not significantly influence the maintenance of porous dry matrix structure, as the sugar abundance (content) in the porous dry matrix increases, the pore volume maintenance rate decreases slightly as in No.10 sample. This demonstrates that the void ratio decreased more than the reduction in void ratio due to pore shrinkage during thermal drying. Hence, it can be thought that sugar in excess of the role of sugar as a binder accumulates in the pores to fill the pores. Therefore, from this result, and with impression on ingestion and water absorption capacity in mind, the amount of sugar as a sweetener used to improve the eating quality of excipient ingredient is appropriately in the range from about 5% to about 40% of the porous dry matrix preparation, also taking the role thereof as a binder into consideration. Provided that the excipient ingredient per se offers a good impression on ingestion, no sugars need to be added in some cases, as is also seen in No.61 wheat flour in Example 13.

Example 5: Effects of choice of sugar or sugar alcohol

The effects of test sweeteners other than white sugar (sorbitol, mannitol, powdered reduced maltose syrup) were investigated. The composition amounts (numbers of grams) listed in Table 8 below were weighed out, and porous dry matrix preparations were prepared in the same manner as Example 2. The void ratio and shape retaining property of the porous dry matrix preparations obtained are shown in Table 8 below.

[Table 8]

|  | No.7 | No.4 | No.11 | No.12 | No.13 |
|---|---|---|---|---|---|
| Carboxymethyl-cellulose sodium | 1.0 | 1.0 | 1.0 | 1.0 | 1.0 |
| Crystalline cellulose | 5.0 | 5.0 | 5.0 | 5.0 | 5.0 |
| Purified water | 15.0 | 15.0 | 15.0 | 15.0 | 15.0 |
| Sugar or sugar alcohol |  | White sugar 4.0 | Powdered reduced maltose syrup 4.0 | Sorbitol 4.0 | Mannitol 4.0 |
| Medium-chain fatty acid triglyceride | 5.0 | 5.0 | 5.0 | 5.0 | 5.0 |
| Amount of water contained before drying | 57.7% | 50.0% | 50.0% | 50.0% | 50.0% |
| Preparation void ratio | 32.0% | 27.8% | 26.5% | 26.7% | 26.1% |
| Shape retaining property | ○ | ○ | ○ | ○ | ○ |
| Impression on ingestion | ○ | ○ | ○ | △ | ○ |
| Water absorption capacity | ○ | ○ | ○ | ○ | ○ |

(continued)

|  | No.7 | No.4 | No.11 | No.12 | No.13 |
|---|---|---|---|---|---|
| After water absorption: Shape retaining property | ○ | ○ | ○ | ○ | ○ |
| Impression on ingestion | ○ | ○ | ○ | ○ | ○ |
| [Note] ○: good, ×: poor, △: equivocal. | | | | | |

Even when powdered reduced maltose syrup, sorbitol, or mannitol was used in place of white sugar, the void ratio was not significantly influenced. From this result, it was demonstrated that irrespective of the choice of sugar or sugar alcohol used, the void ratio remains significantly unchanged. The impression on ingestion was also not significantly influenced.

This result was evaluated by pore volume maintenance rate of dry matrix preparation. The results are shown in Table 9 below. As seen in Table 9, it was demonstrated that the pore volume maintenance rate is not significantly affected by the presence or absence of sugar or sugar alcohol, or the choice of sugar or sugar alcohol.

[Table 9]

|  | No.7 | No.4 | No.11 | No.12 | No.13 |
|---|---|---|---|---|---|
| Choice of sugar | - | White sugar | Maltose | Sorbitol | Mannitol |
| Pore maintenance rate | 55.5% | 55.6% | 53.0% | 53.4% | 52.2% |

Example 6: Effects of medium-chain fatty acid triglyceride as eating quality improving ingredient

To investigate the effects of the amount of medium-chain fatty acid triglyceride added, the composition amounts (numbers of grams) listed in Table 10 below were weighed out, and porous dry matrix preparations were prepared in the same manner as Example 2. The void ratio and shape retaining property of the porous dry matrix preparations obtained are shown in Table 10 below.

[Table 10]

|  | No.2 | No.14 | No.15 | No.16 | No.17 | No.18 | No.4 |
|---|---|---|---|---|---|---|---|
| Carboxymethyl-cellulose sodium | 1.0 | 1.0 | 1.0 | 1.0 | 1.0 | 1.0 | 1.0 |
| Crystalline cellulose | 5.0 | 5.0 | 5.0 | 5.0 | 5.0 | 5.0 | 5.0 |
| Purified water | 15.0 | 15.0 | 15.0 | 15.0 | 15.0 | 15.0 | 15.0 |
| White sugar | 4.0 | 4.0 | 4.0 | 4.0 | 4.0 | 4.0 | 4.0 |
| Medium-chain fatty acid triglyceride | | 1.0 | 2.0 | 3.0 | 3.5 | 4.0 | 5.0 |
| Amount of water contained before drying | 60.0% | 57.7% | 55.6% | 51.7% | 52.6% | 51.7% | 50.0% |
| Void ratio | 42.1% | 35.4% | 34.6% | 31.7% | 29.1% | 28.0% | 27.8% |
| Shape retaining property | ○ | ○ | ○ | ○ | ○ | ○ | ○ |
| Impression on ingestion | ○ | ○ | ○ | ○ | ○ | ○ | ○ |
| Water absorption capacity | ○ | ○ | ○ | ○ | ○ | ○ | ○ |
| After water absorption: Shape retaining property | ○ | ○ | ○ | ○ | ○ | ○ | ○ |

(continued)

|  | No.2 | No.14 | No.15 | No.16 | No.17 | No.18 | No.4 |
|---|---|---|---|---|---|---|---|
| Impression on ingestion | ○ | ○ | ○ | ○ | ○ | ○ | ○ |
| [Note]<br>○: good, ×: poor, Δ: equivocal. | | | | | | | |

From this result, it was found that as the amount of medium-chain fatty acid triglyceride added was increased, the void ratio decreased. In sample No.4, supplemented with 5 g of a medium-chain fatty acid triglyceride, oil oozing out on the preparation surface and other phenomena were observed.

When this result was evaluated by pore volume maintenance rate of porous dry matrix preparation, the results shown in Table 11 below were obtained.

[Table 11]

|  | No.2 | No.14 | No.15 | No.16 | No.17 | No.18 | No.4 |
|---|---|---|---|---|---|---|---|
| Triglyceride content in porous dry matrix preparation | 0% | 9.1% | 16.7% | 23.1% | 25.9% | 28.6% | 33.3% |
| Void ratio | 42.1% | 35.4% | 34.6% | 31.7% | 29.1% | 28.0% | 26.7% |
| Pore volume maintenance rate | 70.2% | 61.4% | 62.2% | 61.3% | 55.3% | 54.2% | 55.6% |
| Triglycerides /excipient ingredient +CMCNa | 0 | 0.17 | 0.33 | 0.5 | 0.58 | 0.67 | 0.83 |
| Shape retaining property | ○ | ○ | ○ | ○ | ○ | ○ | ○ |
| Impression on ingestion | ○ | ○ | ○ | ○ | ○ | ○ | ○ |
| Water absorption capacity | ○ | ○ | ○ | ○ | ○ | ○ | ○ |

As shown by this result, it was shown that in the porous dry matrix preparation formed with this content composition of CMCNa and crystalline cellulose, the pore volume maintenance rate remains within the range from about 61% to about 62% until the medium-chain fatty acid triglyceride content exceeds about 23%, but as the triglyceride content increases to about 26%, the pore volume maintenance rate tends to decrease.

This is thought to be because the triglyceride is used to cover the surface of the porous dry matrix until the triglyceride content exceeds about 23%, and hence the pore volume maintenance rate does not change significantly. It was thought that as the triglyceride content increase to exceed about 26%, the triglyceride in excess increasingly oozes out to the matrix surface and pores.

It is thought that the triglyceride plays a role in conducting heat to the inside of the clay-like composition during thermal drying, and as a result, the water has become likely to evaporate. Hence, it is thought that to form pores communicating with each other to the inside of the dry matrix, it is desirable to add a edible oil/fat (triglyceride).

From these findings, it is thought that to immerse the dry preparation of the present invention in water to prepare a finely textured, sponge-like wet hydrated composition, it is desirable to add a edible oil/fat like triglyceride.

Example 7: Effects of choice of edible oil/fat as impression on ingestion improving ingredient

To investigate the effects of choice of edible oil/fat as impression on ingestion improving ingredient, using corn oil, cottonseed oil, and soy oil in place of a medium-chain fatty acid triglyceride, as with sample No.16, the composition amounts (numbers of grams) listed in Table 12 below were weighed out, and porous dry matrix preparations were prepared in the same manner as Example 2. The void ratio and shape retaining property of the porous dry matrix preparation obtained are shown in Table 12 below.

[Table 12]

|  | No.19 | No.20 | No.21 | No.22 |
|---|---|---|---|---|
| Carboxymethyl-cellulose sodium | 1.0 | 1.0 | 1.0 | 1.0 |

(continued)

| | No.19 | No.20 | No.21 | No.22 |
|---|---|---|---|---|
| Crystalline cellulose | 5.0 | 5.0 | 5.0 | 5.0 |
| Purified water | 15.0 | 15.0 | 15.0 | 15.0 |
| White sugar | 4.0 | 4.0 | 4.0 | 4.0 |
| Edible oil/fat | Medium-chain fatty acid glyceride 3.0 | Corn oil 3.0 | Cotton-seed oil 3.0 | Soy oil 3.0 |
| Amount of water contained before drying | 53.6% | 53.6% | 53.6% | 53.6% |
| Preparation void ratio | 28.6% | 30.3% | 28.9% | 29.5% |
| Shape retaining property | ○ | ○ | ○ | ○ |
| Impression on ingestion | ○ | ○ | ○ | ○ |
| Water absorption capacity | ○ | ○ | ○ | ○ |
| After water absorption: Shape retaining property | ○ | ○ | ○ | ○ |
| Impression on ingestion | ○ | ○ | ○ | ○ |
| [Note]<br>○: good, ×: poor, Δ: equivocal. | | | | |

According to this result, it was found that even when the choice of edible oil/fat was changed, the void ratio was not significantly affected. It was also found that the choice also does not significantly influence water absorption capacity. These results show that edible oil/fat do not have a significant effect on the construction of porous dry matrix, but do contribute mainly to improving the impressions on ingestion of the matrix-forming excipient ingredient, CMCNa and the like.

Example 8: Effect of the amount of purified water added

Regarding the void ratio, which significantly influences impression on ingestion, as shown in the foregoing examples, the rough upper limit of the void ratio depends on the amount of water contained before drying and, when the water evaporates during thermal drying, settles to a certain level while the space left after evaporation of the water shrinking to some extent. For this reason, as the amount of water contained increases, the void ratio tends to increase, but it becomes more likely that a rough matrix is formed, and a dry matrix with uniform pores tends to be unlikely to be formed. In a porous dry matrix preparation wherein the amount of water contained is large, shape retaining property worsens, and if further water is absorbed, the preparation tends to often collapse into pieces. Hence, the effects of the amount of water contained on porous dry matrix structure formation were determined. For this purpose, the ingredients listed in Table 13 below were weighed out, the amount of purified water was changed, and porous dry matrix preparations were prepared in the same manner as Example 2. The void ratio and shape retaining property of the porous dry matrix preparations obtained are shown in Table 13 below.
[Table 13]

| | No.23 | No.24 | No.25 | No.26 | No.27 |
|---|---|---|---|---|---|
| Carboxymethyl-cellulose sodium | 1.0 | 1.0 | 1.0 | 1.0 | 1.0 |
| Crystalline cellulose | 5.0 | 5.0 | 5.0 | 5.0 | 5.0 |
| Purified water | 5.0 | 7.0 | 10.0 | 15.0 | 20.0 |
| White sugar | 4.0 | 4.0 | 4.0 | 4.0 | 4.0 |
| Medium-chain fatty acid triglyceride | 5.0 | 5.0 | 5.0 | 5.0 | 5.0 |
| Amount of water contained before drying | 25.0% | 31.8% | 40.0% | 50.0% | 57.1% |

EP 2 119 454 A1

(continued)

|  | No.23 | No.24 | No.25 | No.26 | No.27 |
|---|---|---|---|---|---|
| Preparation void ratio | 19.5% | 19.9% | 21.5% | 26.7% | 28.5% |
| Shape retaining property | ○ | ○ | ○ | ○ | ○ |
| Impression on ingestion | ○ | ○ | ○ | ○ | ○ |
| Water absorption capacity | ○ | ○ | ○ | ○ | ○ |
| After water absorption: Shape retaining property | × | Δ | ○ | ○ | ○ |
| Impression on ingestion | × | Δ | ○ | ○ | ○ |
| [Note]<br>○: good, ×: poor, Δ: equivocal. | | | | | |

As shown by this result, even when the porous dry matrix preparation composition after drying is constant, it was shown that the amount of water contained in a mixed composition before drying increases, the void ratio increases. Meanwhile, it was shown that when the amount of water contained decreases, and the void ratio decreases below about 20%, shape retaining property after water absorption worsens. Therefore, it was found that the amount of water contained is desirably higher than about 25%, more preferably an amount of water contained of not less than about 30% is effective in securing an appropriate void ratio.
When these results were evaluated by pore volume maintenance rate and the content of excipient ingredient and the like (crystalline cellulose+CMCNa) before drying, the results shown in Table 14 below were obtained.
[Table 14]

|  | No.23 | No.24 | No.25 | No.26 | No.27 |
|---|---|---|---|---|---|
| Amount of water contained before drying | 25.0% | 31.8% | 40.0% | 50.0% | 57.1% |
| Pore volume maintenance rate | 78.0% | 62.6% | 53.8% | 53.4% | 49.9% |
| Content of excipient ingredient and the like (crystalline cellulose+CMC Na) | 30.0% | 27.3% | 24.0% | 20.0% | 17.1% |
| After water absorption: Shape retaining property | × | Δ | ○ | ○ | ○ |
| Impression on ingestion | × | Δ | ○ | ○ | ○ |

As shown by this result, as the amount of water contained in a mixed composition before drying decreases, the void ratio decreases proportionally; meanwhile, the pore volume maintenance rate tends to increase.
This finding indicates that because as the amount of water contained decreases, the content of excipient ingredient and the like in a mixed composition before drying increases relatively, the amount of the excipient ingredient and the like that support the pores increases relatively. As a result, pore shrinkage during drying is suppressed. Hence, when the amount of water contained is low, a high pore volume maintenance rate is obtained.

Example 9: Effects of change in the amount of excipient ingredient added on void ratio

As shown in Example 8, it was demonstrated that the upper limit of the void ratio depends roughly on the amount of water contained, and the pore volume maintenance rate is significantly influenced by the content of excipient ingredient and the like. Hence, with sample No.26, which has a void ratio of about 27%, being the reference, the effects of excipient ingredients in a range of the amount of water contained of around 50% were investigated. The ingredients in the composition listed in the table below were weighed out, and porous dry matrix preparations were prepared in the same manner as Example 2. The void ratio and shape retaining property of the porous dry matrix preparations obtained are shown in Table 15 below.
[Table 15]

| | No.26 | No.28 | No.29 | No.30 | No.31 | No.32 | No.33 |
|---|---|---|---|---|---|---|---|
| Carboxymethyl-cellulose sodium | 1.0 | 1.0 | 1.0 | 1.0 | 1.0 | 1.0 | 1.0 |
| Crystalline cellulose | 5.0 | 5.0 | 7.0 | 7.2 | 7.2 | 7.4 | 7.4 |
| Purified water | 15.0 | 15.0 | 15.0 | 15.0 | 15.0 | 15.0 | 15.0 |
| White sugar | 4.0 | 4.0 | 4.0 | 4.0 | 4.0 | 4.0 | 4.0 |
| Medium-chain fatty acid triglyceride | 5.0 | 3.5 | 3.5 | 3.5 | 3.6 | 3.5 | 3.7 |
| Amount of water contained before drying | 50.0% | 52.6% | 49.2% | 48.9% | 48.7% | 48.5% | 48.2% |
| Preparation void ratio | 26.7% | 29.1% | 28.2% | 28.2% | 30.4% | 29.4% | 29.8% |
| Shape retaining property | ○ | ○ | ○ | ○ | ○ | ○ | ○ |
| Impression on ingestion | ○ | ○ | ○ | ○ | ○ | ○ | ○ |
| Water absorption capacity | ○ | ○ | ○ | ○ | ○ | ○ | ○ |
| After water absorption: Shape retaining property | ○ | ○ | ○ | ○ | ○ | ○ | ○ |
| Impression on ingestion | ○ | ○ | ○ | ○ | ○ | ○ | ○ |
| [Note]<br>○: good, ×: poor, Δ: equivocal. | | | | | | | |

From this result, it was found that if the amount of water contained is around 50%, as is evident from a comparison of sample No.28 and sample No.32, even when the excipient ingredient was increased by about 50%, the void ratio was little influenced. Therefore, it was shown that when the amount of water contained was around 50%, the excipient ingredient did not influence matrix formation because of the abundance of water.

When these results were evaluated by pore volume maintenance rate and the content of excipient ingredient and the like (crystalline cellulose+CMCNa) before drying, the results shown in Table 16 below were obtained.

[Table 16]

| | No.26 | No.28 | No.29 | No.30 | No.31 | No.32 | No.33 |
|---|---|---|---|---|---|---|---|
| Preparation Void ratio | 26.7% | 29.1% | 28.2% | 28.2% | 30.4% | 29.4% | 29.8% |
| Crystalline cellulose (g) | 5.0 | 5.0 | 7.0 | 7.2 | 7.2 | 7.4 | 7.4 |
| Pore volume maintenance rate | 53.4% | 55.3% | 57.3% | 57.7% | 62.4% | 60.6% | 61.8% |
| Content of excipient ingredient and the like (Crystalline cellulose+CM CNa) | 20.0% | 21.1% | 26.2% | 26.7% | 26.6% | 27.2% | 27.0% |
| Shape retaining property | ○ | ○ | ○ | ○ | ○ | ○ | ○ |
| Impression on ingestion | ○ | ○ | ○ | ○ | ○ | ○ | ○ |

According to this result, it was found that as the excipient ingredient increases by about 50%, the pore volume maintenance rate improves slightly. Furthermore, comparing the results from sample Nos.24 and 25 in Example 8 with the result in Table 16, it was found that as the content of the excipient ingredient and the like (crystalline cellulose+CMCNa) in the dry preparation exceeds about 27%, the pore volume maintenance rate exceeds about 60%.

Hence, it was shown that to suppress pore shrinkage during drying to ensure the formation of an appropriate porous dry matrix preparation, increasing the content of excipient ingredient and the like (crystalline cellulose+CMCNa) in the mixed composition to some extent is effective.

Example 10: Effects of the amount of carboxymethylcellulose salt added as structure ingredient

As shown in Example 9, it was found that provided that the amount of water contained was around 50%, even when the excipient ingredient was increased by about 50%, the void ratio was little influenced. It was also shown that as the content of excipient ingredient and the like (crystalline cellulose+CMCNa) exceeds about 27%, the pore volume maintenance rate exceeds about 60%. Hence, in the presence of the same amount of water contained, the influence of changes in the content of excipient ingredient and the like (crystalline cellulose+CMCNa) was investigated. Particularly, focusing on the composition of sample No.31, which had a good pore volume maintenance rate of 62.4%, the amount of CMCNa added was changed, and changes in the void ratio and pore volume maintenance rate were investigated. For this purpose, the composition amounts (numbers of grams) listed in Table 17 below were weighed out, and porous dry matrix preparations were prepared in the same manner as Example 2. The void ratio and the like of the porous dry matrix preparation obtained are shown in Table 17 below.

[Table 17]

| | No.34 | No.35 | No. 36 | No. 37 | No. 38 | No.39 | No.40 | No.41 | No.31 | No.42 | No.43 |
|---|---|---|---|---|---|---|---|---|---|---|---|
| Carboxymethyl-cellulose sodium | 0.072 | 0.144 | 0.16 | 0.18 | 0.24 | 0.36 | 0.72 | 0.90 | 1.00 | 1.44 | 2.40 |
| Crystalline cellulose | 7.20 | 7.20 | 7.20 | 7.20 | 7.20 | 7.20 | 7.20 | 7.20 | 7.20 | 7.20 | 7.20 |
| Purified water | 15.0 | 15.0 | 15.0 | 15.0 | 15.0 | 15.0 | 15.0 | 15.0 | 15.0 | 15.0 | 15.0 |
| Powdered reduced maltose syrup | 4.0 | 4.0 | 4.0 | 4.0 | 4.0 | 4.0 | 4.0 | 4.0 | 4.0 | 4.0 | 4.0 |
| Medium-chain fatty acid triglyceride | 3.60 | 3.60 | 3.60 | 3.60 | 3.60 | 3.60 | 3.60 | 3.60 | 3.60 | 3.60 | 3.60 |
| Amount of water contained (%) before drying | 50.2 | 50.1 | 50.1 | 50.0 | 49.9 | 49.7 | 49.1 | 48.9 | 48.7 | 48.0 | 46.6 |
| Preparation void ratio (%) | 38.4 | 39.2 | 39.9 | 39.7 | 47.3 | 44.5 | 34.5 | 31.6 | 30.4 | 29.6 | 25.8 |
| Shape retaining property | × | Δ | Δ | Δ | Δ | ○ | ○ | ○ | ○ | ○ | ○ |
| Impression on ingestion | ○ | ○ | ○ | ○ | ○ | ○ | ○ | ○ | ○ | ○ | Δ |
| Water absorption capacity | ○ | ○ | ○ | ○ | ○ | ○ | ○ | Δ | ○ | Δ | × |
| Shape retaining property after water absorption | Δ | Δ | ○ | ○ | ○ | ○ | ○ | ○ | ○ | ○ | × Brittle |
| Impression on ingestion after water absorption | Δ | ○ | ○ | ○ | ○ | ○ | ○ | ○ | ○ | ○ | Δ |
| [Note] ○: good, ×: poor, Δ: equivocal. | | | | | | | | | | | |

As shown by this result, it was found that as the amount of CMCNa added increases, the void ratio changes on a bell-shaped curve with that of sample No.38 being a peak. Hence, it was found that when the amount of water contained was around about 50%, an amount of excipient ingredient and the like (crystalline cellulose+CMCNa) added in a certain range maximizes the void ratio.

To determine whether the content of CMCNa out of excipient ingredient and the like (crystalline cellulose+CMCNa) is an important factor, or the content of excipient ingredient crystalline cellulose is important, a reevaluation was performed by pore volume maintenance rate of porous dry matrix preparation. The results are shown in Table 18 below.

[Table 18]

| | No. 34 | No. 35 | No. 36 | No. 37 | No. 38 | No. 39 | No. 40 | No. 41 | No. 31 | No. 42 | No. 43 |
|---|---|---|---|---|---|---|---|---|---|---|---|
| Pore volume maintenance rate (%) | 76.5 | 78.2 | 79.6 | 79.7 | 94.8 | 89.5 | 70.3 | 64.6 | 62.4 | 61.7 | 55.6 |
| Content of excipient ingredient and the like (crystalline cellulose+CMCNa) (%) | 24.3 | 24.5 | 24.6 | 24.6 | 24.8 | 25.1 | 26.0 | 26.4 | 26.6 | 27.7 | 29.8 |
| Excipient ingredient/CMCNa | 100 | 50 | 45 | 40 | 30 | 20 | 10 | 8 | 7.2 | 5 | 3 |
| Shape retaining property | × | Δ | Δ | Δ | Δ | ○ | ○ | ○ | ○ | ○ | ○ |
| Impression on ingestion | ○ | ○ | ○ | ○ | ○ | ○ | ○ | ○ | ○ | ○ | Δ |

It is shown in Example 8 and Example 9 that as the content of excipient ingredient and the like (crystalline cellulose+CMCNa) increases, the pore volume maintenance rate tends to improve. Meanwhile, according to the results in Table 18, it was shown that as the content of excipient ingredient and the like (crystalline cellulose+CMCNa) before drying increases, the pore volume maintenance rate changes on a bell-shaped curve. It should be noted, however, that in the results in Table 18, the amount of crystalline cellulose was constant at 7.20 g, and only the amount of CMCNa changed. For example, in sample No.34, wherein the amount of CMCNa is about 1/10 of the amount of CMCNa in sample No.31, the reference for composition, a high pore volume maintenance rate of about 77%, exceeding about 62% for No.31, was obtained.

This result in Table 18 shows that there is a suitable composition ratio for matrix formation between excipient ingredient (crystalline cellulose) and structure ingredient (CMCNa). For example, as in sample No.38, when the quantitative ratio of excipient ingredient (crystalline cellulose) and structure ingredient (CMCNa) was around about 30:1, the maximum pore volume maintenance rate was obtained.

This indicates that any composition ratio around this level suitably enables the formation of porous dry matrix structure. Also, it is shown that a matrix having a strength that endures pore shrinkage during drying can be formed. Judging from this result, it is seen that in the case of sample No.43, the amount of CMCNa is too much for the formation of a porous dry matrix; it was thought that the excess amount of CMCNa acts to fill the pores, resulting in a reduction in the pore volume maintenance rate. Meanwhile, in the case of sample No.34, it is thought that because the amount of CMCNa is insufficient, the shape retaining property of the porous dry matrix preparation has worsened.

From these findings, it is evident that to construct an appropriate porous dry matrix, an appropriate ratio by mass of excipient ingredient (crystalline cellulose) and structure ingredient (CMCNa) is important. Hence, it was found that regarding the ratio by mass of structure ingredient (CMCNa) and excipient ingredient, assuming the amount of structure ingredient (CMCNa) to be 1 part by mass, taking shape retaining property into consideration, it is desirable that the amount of excipient ingredient (crystalline cellulose) be in the range from not less than about 3 parts by mass to less than 100 parts by mass.

To achieve an improvement in the pore volume maintenance rate, it can be said that for example, to obtain a pore volume maintenance rate exceeding about 70%, the content ratio of excipient ingredient (crystalline cellulose) is preferably in the range from not less than about 10 to not more than 100.

Hence, since it was found that changes in the amount of CMCNa added significantly influence the pore volume maintenance rate, the CMCNa content and the content of excipient ingredient and the like (crystalline cellulose and CMCNa) in the porous dry matrix preparation were compared, and the effects on pore volume maintenance rate were rated. The results are shown in Table 19 below.

[Table 19]

|  | No.34 | No.35 | No.36 | No.37 | No.38 | No.39 | No.40 | No.41 | No.31 | No.42 | No.43 |
|---|---|---|---|---|---|---|---|---|---|---|---|
| Pore volume maintenance rate (%) | 76.5 | 78.2 | 79.6 | 79.7 | 94.8 | 89.5 | 70.3 | 64.6 | 63.7 | 61.7 | 55.6 |
| CMCNa content (%) in dry matrix preparation | 0.5 | 1.0 | 1.1 | 1.2 | 1.6 | 2.4 | 4.6 | 5.7 | 6.3 | 8.9 | 14.0 |
| Excipient ingredient content (%) in dry matrix preparation | 48.4 | 48.2 | 48.1 | 48.1 | 47.9 | 47.5 | 46.4 | 45.9 | 45.6 | 44.3 | 41.9 |
| Shape retaining property | × | △ | △ | △ | △ | ○ | ○ | ○ | ○ | ○ | ○ |
| Impression on ingestion | ○ | ○ | ○ | ○ | ○ | ○ | ○ | ○ | ○ | ○ | △ |

As shown by this result, the effect on pore volume maintenance rate is significantly influenced by changes in the amount of CMCNa added. Taking the impression on ingestion of the porous dry matrix preparation into consideration, it is desirable that the amount of CMCNa added be in the range from about 0.5% to about 14.0% in the dry matrix. Taking shape retaining property into consideration, it was found that a suitable amount of CMCNa added for the construction of a porous dry matrix is in the range from about 1% to about 14%.

This suitable amount of CMCNa added is observed in compositions with a content of excipient ingredient (crystalline cellulose) and the like similar to that in sample No.31, wherein the amount of water contained is around about 50%. Hence, factors that influence the formation of porous dry matrix structure were further investigated.

Example 11: Effects of changes in the amount of water on the void ratio of porous dry matrix preparation

Since a suitable range of CMCNa content was demonstrated in a mixed composition wherein the amount of water contained is around about 50% in Example 10, using the composition of sample No.39 (a composition of excipient ingredient and CMCNa suitable for formation of porous dry matrix preparation), the amount of water contained was changed, and the effects of the ratio of the amounts of water and excipient and the like (crystalline cellulose+CMCNa) were evaluated and investigated. The composition amounts (numbers of grams) listed in Table 20 below were weighed out, and porous dry matrix preparations were prepared in the same manner as Example 2. The void ratio and shape retaining property of the porous dry matrix preparation obtained are shown in Table 20 below.

[Table 20]

|  | No.44 | No.45 | No.46 | No.47 | No.39 | No.48 | No.49 | No.50 |
|---|---|---|---|---|---|---|---|---|
| Carboxymethylcellulose sodium | 0.36 | 0.36 | 0.36 | 0.36 | 0.36 | 0.36 | 0.36 | 0.36 |
| Crystalline cellulose | 7.20 | 7.20 | 7.20 | 7.20 | 7.20 | 7.20 | 7.20 | 7.20 |
| Purified water | 5.0 | 7.0 | 10.0 | 13.0 | 15.0 | 18.0 | 20.0 | 23.0 |
| Powdered reduced maltose syrup | 4.0 | 4.0 | 4.0 | 4.0 | 4.0 | 4.0 | 4.0 | 4.0 |
| Medium-chain fatty acid triglyceride | 3.60 | 3.60 | 3.60 | 3.60 | 3.60 | 3.60 | 3.60 | 3.60 |

(continued)

| | No.44 | No.45 | No.46 | No.47 | No.39 | No.48 | No.49 | No.50 |
|---|---|---|---|---|---|---|---|---|
| Amount of water contained (%) before drying | 24.8 | 31.6 | 39.8 | 46.2 | 49.7 | 54.3 | 56.9 | 60.3 |
| Preparation void ratio(%) | 20.8 | 26.6 | 34.4 | 39.1 | 44.5 | 47.9 | 47.3 | 43.1 |
| Shape retaining property | ○ | ○ | ○ | ○ | ○ | Δ | Δ | × |
| Impression on ingestion | × | ○ | ○ | ○ | ○ | ○ | Δ | Δ |
| Water absorption capacity | × | Δ | Δ | ○ | ○ | ○ | ○ | ○ |
| Shape retaining property after water absorption | ○ | ○ | ○ | ○ | ○ | ○ | ○ | Δ |
| Impression on ingestion after water absorption | Δ | ○ | ○ | ○ | ○ | ○ | ○ | ○ |
| [Note]<br>○: good, ×: poor, Δ: equivocal. | | | | | | | | |

From this result, it was found that as the amount of water contained increases, the void ratio changes on a bell-shaped curve with that of sample No.48 being a peak. Since the increase in void ratio is slightly slowed down as the amount of water contained increases, it is thought that if the amount of water contained exceeds 60% as shown in sample No.50, pore-forming materials of excipient ingredient and the like are lacked, so that it is difficult to retain the pores after evaporation of water.

When these results are expressed by changes in the amount of water contained and the pore volume maintenance rate of porous dry matrix preparation, the results shown in Table 21 below are obtained.

[Table 21]

| | No.44 | No.45 | No.46 | No.47 | No.39 | No.48 | No.49 | No.50 |
|---|---|---|---|---|---|---|---|---|
| Pore volume maintenance rate (%) | 83.9 | 84.2 | 86.4 | 84.6 | 89.5 | 88.2 | 83.1 | 71.5 |
| Content of excipient ingredient and the like (crystalline cellulose+CMCNa) before drying (%) | 37.5 | 34.1 | 30.0 | 26.8 | 25.1 | 22.8 | 21.5 | 19.8 |
| Amount of water contained before drying (%) | 24.8 | 31.6 | 39.8 | 46.2 | 49.7 | 54.3 | 56.9 | 60.3 |
| Shape retaining property | ○ | ○ | ○ | ○ | ○ | Δ | Δ | × |
| Impression on ingestion | × | ○ | ○ | ○ | ○ | ○ | Δ | Δ |

As shown in Table 21, it was shown that the pore volume maintenance rate was in an almost constant range (about 84 to about 90%) when the amount of water contained was in the broad range from about 25% to about 57%. This means that the composition of sample No.39 (a composition of excipient ingredient and CMCNa that is suitable for the formation of porous dry matrix preparation) is not easily influenced by changes in the water content.

Therefore, it was found that in the appropriate composition range (range of quantitative ratio of CMCNa and excipient ingredient) for the formation of a porous dry matrix preparation, by increasing the amount of water contained in the mixed composition before drying, the void ratio can be improved.

Taking the pore volume maintenance rate into consideration, it is seen that to form an appropriate porous dry matrix structure that endures pore shrinkage during drying, not only the CMCNa content, but also the excipient ingredient content is important. As the content of CMCNa and excipient ingredient before drying, taking shape retaining property into consideration, not less than about 20% is preferable; judging from available data, the content is suitably in the range from about 20% to about 45%.

Taking the shape retaining property and impression on ingestion (tooth touch based on void ratio) of the porous dry matrix preparation into consideration, it was shown that as with the results in Example 8, an amount of water contained such that the void ratio is not less than about 20% is appropriate, and that the amount of water contained in the composition before drying of not less than about 25% is desirable therefor. Taking the shape retaining property of porous dry matrix preparation into consideration, it was found that the amount of water contained is more suitably in the range from about 30% to about 57%.

Example 12: Effects of the amount of excipient ingredient (crystalline cellulose) added

In Example 10, the appropriate composition range (range of quantitative ratio of CMCNa and excipient ingredient) for the formation of a porous dry matrix preparation became evident, and it was shown that in the range, as is evident in Example 11, the preparation is unlikely to be influenced by the changes in the amount of water contained. Hence, focusing on sample No.39, the composition ratio of excipient ingredient (crystalline cellulose) was widely changed, and the effects of the amount of excipient ingredient added on void ratio and pore volume maintenance rate were investigated. Samples in the composition amounts (numbers of grams) listed in Table 22 below were weighed out, and then porous dry matrix preparations were prepared in the same manner as Example 2. The void ratio and shape retaining property of the porous dry matrix preparations obtained are shown in Table 22 below.

[Table 22]

|  | No.51 | No.52 | No.53 | No.54 | No.39 | No.55 | No.56 | No.57 | No.58 |
|---|---|---|---|---|---|---|---|---|---|
| Carboxymethylcellulose sodium | 0.36 | 0.36 | 0.36 | 0.36 | 0.36 | 0.36 | 0.36 | 0.36 | 0.36 |
| Crystalline cellulose | 1.80 | 3.60 | 4.32 | 5.40 | 7.20 | 9.00 | 10.80 | 14.40 | 18.0 |
| Purified water | 15.0 | 15.0 | 15.0 | 15.0 | 15.0 | 15.0 | 15.0 | 15.0 | 15.0 |
| Powdered reduced maltose syrup | 4.0 | 4.0 | 4.0 | 4.0 | 4.0 | 4.0 | 4.0 | 4.0 | 4.0 |
| Medium-chain fatty acid triglyceride | 3.60 | 3.60 | 3.60 | 3.60 | 3.60 | 3.60 | 3.60 | 3.60 | 3.60 |
| Amount of water contained before drying (%) | 60.6 | 56.5 | 55.0 | 52.9 | 49.7 | 46.9 | 44.4 | 40.1 | 36.6 |
| Preparation void ratio (%) | 21.5 | 31.7 | 35.6 | 44.0 | 44.5 | 43.0 | 40.3 | 38.8 | 36.5 |
| Shape retaining property | × | △ | △ | △ | ○ | ○ | ○ | ○ | ○ |
| Impression on ingestion | × | ○ | ○ | ○ | ○ | ○ | △ | △ | △ |
| Water absorption capacity | △ | ○ | ○ | ○ | ○ | ○ | × | × | × |
| Shape retaining property after water absorption | × | ○ | △ | ○ | ○ | ○ | - | - | - |
| Impression on ingestion after water absorption | △ | ○ | ○ | △ | ○ | ○ | - | - | - |
| [Note] ○: good, ×: poor, △: equivocal. -: Not evaluable because of poor water absorption capacity. | | | | | | | | | |

From the above-described results, it was demonstrated that as the amount of crystalline cellulose added increases, the void ratio changes on a bell-shaped curve with that of sample No.39 being a peak.
When this result was evaluated by pore volume maintenance rate and the content of excipient ingredient and the like before drying, the results shown in Table 23 below are obtained.
[Table 23]

|  | No.51 | No.52 | No.53 | No.54 | No.39 | No.55 | No.56 | No.57 | No.58 |
|---|---|---|---|---|---|---|---|---|---|
| Pore volume maintenance rate (%) | 35.5 | 56.1 | 64.7 | 83.2 | 89.5 | 91.7 | 90.8 | 96.8 | 99.7 |

(continued)

|  | No.51 | No.52 | No.53 | No.54 | No.39 | No.55 | No.56 | No.57 | No.58 |
|---|---|---|---|---|---|---|---|---|---|
| Content of excipient ingredient and the like (Crystalline cellulose+CMCNa) before drying (%) | 8.7 | 14.9 | 17.7 | 20.3 | 25.1 | 29.3 | 33.1 | 39.5 | 44.8 |
| Excipient ingredient/CMCNa | 5 | 10 | 12 | 15 | 20 | 25 | 30 | 40 | 50 |
| CMCNa content in preparation (%) | 3.7 | 3.1 | 2.9 | 2.7 | 2.4 | 2.1 | 1.9 | 1.6 | 1.4 |
| Crystalline cellulose content in preparation | 18.4 | 31.1 | 35.2 | 40.4 | 47.5 | 53.1 | 57.6 | 64.4 | 69.3 |
| Shape retaining property | × | Δ | Δ | Δ | ○ | ○ | ○ | ○ | ○ |
| Impression on ingestion | × | ○ | ○ | ○ | ○ | ○ | Δ | Δ | Δ |

According to the results in Table 23, the same results as Example 10 were obtained. Specifically, it is seen that in terms of pore volume maintenance rate, to form an appropriate dry matrix structure that endures pore shrinkage during drying, not only the CMCNa content, but also the excipient ingredient content is important. Regarding the content of a mixed composition of CMCNa and excipient ingredient before drying, taking shape retaining property and impression on ingestion into consideration, it is seen that the content is suitably in the range from about 15% to about 45%. The quantitative ratio of excipient ingredient and CMCNa is also important, and the quantitative ratio (excipient ingredient/ CMCNa) is suitably not less than about 10; taking the results in Example 10 into consideration, it was shown that the ratio is in the range from about 10 to about 100. Furthermore, it is suggested that the CMCNa content in the porous dry matrix preparation is preferably in the range below about 3.7%.

As the excipient ingredient content increases, the matrix structure becomes firmer, and shape retaining property and pore maintenance rate improve significantly. It should be noted, however, that problems can arise with respect to shape retaining property and impression on ingestion as the excipient ingredient content increases, which in turn can render the porous dry matrix preparation hard and floury, and can worsen the water absorption capacity of the porous dry matrix preparation.

From these results, the content of excipient ingredient in the porous dry matrix preparation is preferable not less than about 30%, and taking shape retaining property into consideration, the content is preferably in the range from about 30% to about 80%.

If the content of excipient ingredient is to be increased, a sweetener and edible oil/fat are sometimes added to the above-described composition or used in combination in order to further improve the impression on ingestion. To increase water absorption capacity, addition of a pH regulator and the like is sometimes necessary. It was found that with the contents of excipient ingredient and CMCNa, and the quantitative ratios of excipient ingredient and CMCNa, of porous dry matrix preparations that have been obtained so far as the base composition, by choosing appropriate additives according to the impression on ingestion and properties of the excipient ingredient, or according to the content composition, and finely adjusting the composition ratio, a desired porous dry matrix preparation can be obtained.

Example 13: Effects of addition of various excipient ingredients

To verify the results in Example 12, crystalline cellulose was replaced with other excipient ingredient, and the effects of various excipient ingredients replaced were evaluated. The ingredients listed in Table 24 below were weighed out, and preparations were prepared in the same manner as Example 2. The void ratio and shape retaining property of the dry preparations obtained are shown in Table 24 below.

[Table 24]

|  | No.39 | No.59 | No.60 | No.61 |
|---|---|---|---|---|
| Carboxymethylcellulose sodium | 0.36 | 0.36 | 0.36 | 1.0 |
| Excipient ingredient | Crystalline cellulose 7.20 | Kaolin 9.10 | Activated charcoal 9.60 | wheat flour 10.0 |

(continued)

|  | No.39 | No.59 | No.60 | No.61 |
|---|---|---|---|---|
| Purified water | 15.0 | 15.0 | 15.0 | 30.0 |
| Powdered reduced maltose syrup | 4.0 | 4.0 | 4.0 | - |
| Medium-chain fatty acid triglyceride | 3.60 | 3.60 | 3.60 | 5.0 |
| Amount of water contained before drying | 49.7% | 46.8% | 46.1% | 66.1% |
| Preparation void ratio | 44.5% | 43.2% | 46.0% | 46.9% |
| Shape retaining property | ○ | ○ | ○ | ○ |
| Impression on ingestion | ○ | ○ | ○ | ○ |
| Water absorption capacity | ○ | ○ | ○ | ○ |
| After water absorption: Shape retaining property | ○ | ○ | ○ | ○ |
| Impression on ingestion | ○ | ○ | Δ | ○ |
| [Note] ○: good, ×: poor, Δ: equivocal. | | | | |

From this result, it was found that for example, a powder that is almost insoluble in water, such as crystalline cellulose, kaolin, activated charcoal and wheat flour, can be used as the excipient ingredient, the void ratio of the porous dry matrix preparation is little influenced by their choice.

When this result was evaluated by pore volume maintenance rate and the content of excipient ingredient and the like, the results shown in Table 25 below were obtained.

[Table 25]

|  | No.39 | No.59 | No.60 | No.61 |
|---|---|---|---|---|
| Pore volume maintenance rate (%) | 89.5 | 92.3 | 99.7 | 71.0 |
| Excipient ingredient/CMCNa | Crystalline cellulose 20 | Kaolin 25.3 | Activated charcoal 26.7 | Wheat flour 10 |
| CMCNa content in preparation | 2.4% | 2.1% | 2.1% | 6.3% |
| Excipient ingredient content in preparation | 47.5% | 53.3% | 54.7% | 62.5% |
| Shape retaining property | ○ | ○ | ○ | ○ |
| Impression on ingestion | ○ | ○ | ○ | ○ |
| Water absorption capacity | ○ | ○ | ○ | ○ |

From this result, it was found that as shown in Example 12, a porous dry matrix preparation that shows a similarly good void ratio and pore volume maintenance rate was obtained even when the excipient ingredient was replaced over a range close to the composition ratio of sample No.39, which employs crystalline cellulose as the excipient ingredient. It was also found that this dry matrix preparation also had good shape retaining property, impression on ingestion, and water absorption capacity.

As shown in sample No.61, it was demonstrated that when the excipient ingredient is wheat flour, because the impression on ingestion of the excipient ingredient is good, a porous dry matrix preparation with a good impression on ingestion can be prepared without adding a sugar or a sugar alcohol as the sweetener.

Example 14: Effects of addition of various excipient ingredients

Replacing with various excipient ingredients, their effects were evaluated. Specifically, using water-soluble powders (hydroxypropylcellulose and polyvinylpyrrolidone) in place of a powder that is almost insoluble in water, making of a porous dry matrix preparation was investigated. Hence, as described above, like sample No.39, the ingredients listed in Table 26 below were weighed out, and porous dry matrix preparations were prepared in the same manner as Example 2. The void ratio and shape retaining property of the porous dry matrix preparations obtained are shown in Table 26 below.

[Table 26]

|  | No.62 | No.63 | No.64 |
|---|---|---|---|
| Carboxymethyl-cellulose sodium | 0.36 | 0.36 | 0.36 |
| Excipient ingredient | Hydroxypropyl-cellulose 7.20 | Polyvinyl-pyrrolidone 7.2 | Cornstarch 7.2 |
| Purified water | 15.0 | 15.0 | 15.0 |
| Powdered reduced maltose syrup | 4.0 | 4.0 | 4.0 |
| Medium-chain fatty acid triglyceride | 3.60 | 3.60 | 3.60 |
| Amount of water contained before drying | 49.7% | 49.7% | 49.7% |
| Preparation void ratio | 25.9% | 5.4% | 41.4% 150˚C one-face baking rod molding |
| Shape retaining property | Nonuniform pores | ○ | ○ |
| Impression on ingestion | × | ○ | ○ |
| Water absorption capacity | × | × | ○ |
| After water absorption: Shape retaining property | × | × | ○ |
| Impression on ingestion | × | × | ○ |
| [Note] ○: good, ×: poor, Δ: equivocal. | | | |

As shown by this result, in the case of an excipient ingredient that dissolves in water, as exemplified by hydroxypropylcellulose in sample No.62, even though it is a cellulose-series derivative, in the case of a water-soluble powder, no porous dry matrix preparation having uniform pores was produced. The dry matrix preparation obtained had slightly rough pores, and the properties thereof, such as shape retaining property and impression on ingestion, were poor. Likewise, as shown in the case of polyvinylpyrrolidone in sample No.63 as the excipient ingredient that dissolves in water, the preparation does not have a sufficient void ratio. Therefore, this preparation does not fall in the scope of the porous dry matrix preparation of the present invention.

Meanwhile, it was found that in the case of a powder that is almost insoluble in water, as exemplified by cornstarch in sample No.64, a similarly good porous dry matrix preparation can be prepared with the same composition as sample No.39.

Like this, from the results of Example 13 and Example 14, it was shown that when a powder that is almost insoluble in water is used as excipient ingredient, a porous dry matrix preparation can be formed irrespective of the choice thereof.

Example 15: Investigation of polystyrenesulfonate-containing porous dry matrix preparation

Based on the findings obtained thus far, preparing a polystyrenesulfonate preparation was investigated. First, since daily ingestion dose is up to 30 g, a preparation was formulated using polystyrenesulfonate as the excipient ingredient, and an attempt was made to increase the content thereof to the maximum possible extent. The polystyrenesulfonates used in the present invention were Calcium Polystyrenesulfonate and Sodium Polystyrenesulfonate specified in the Japanese Pharmacopoeia.

(1) Porous dry matrix preparation of sodium polystyrenesulfonate and calcium polystyrenesulfonate

Taking the result of Example 12 into consideration, preparing a preparation wherein the polystyrenesulfonate content in the dry preparation was around about 60% was investigated. Hence, the ingredients (numbers of grams) listed in Table 27 below were weighed out, and dry preparations were prepared in the same manner as Example 2. From the results shown in Table 27, it was found that even when the excipient ingredient content is high, a preparation having excellent shape retaining property and impression on ingestion is obtained.
[Table 27]

|  | No.65 | No.66 |
|---|---|---|
| Polystyrenesulfonate | Sodium salt: 25 | Calcium salt: 25 |
| Carboxymethylcellulose sodium | 0.6 | 0.6 |
| D-sorbitol | 8.0 | 8.0 |
| Saccharin sodium | 0.02 | 0.04 |
| Medium-chain fatty acid triglyceride | 7.0 | 7.0 |
| Purified water | 28 | 25 |
| Total | 68.62 g | 65.64 g |
| Excipient ingredient content in dry preparation (%) | 61.5 | 61.5 |
| Excipient ingredient/CMCNa | 41.7 | 41.7 |
| Shape retaining property | ○ | ○ |
| Impression on ingestion | ○ | ○ |
| [Note]<br>○: good, ×: poor, Δ: equivocal. | | |

(2) Evaluation test for improvement of water absorption capacity by addition of pH regulator

A pH regulator was added, and the effects on changes in water absorption capacity and the impression on ingestion of the hydrated composition after re-impregnation with water were evaluated. Hence, the reagents (numbers of grams) listed in Table 28 below were weighed out, and porous dry matrix preparations were prepared in the same manner as Example 2. The results are shown in Table 28. As a general tendency, it was found that when citric acid or sodium citrate was added as the pH regulator as described above, water absorption capacity and the impression on ingestion of hydrated composition after re-impregnation with water improve.
[Table 28]

|  | No.67 | No.68 | No.69 | No.70 |
|---|---|---|---|---|
| Calcium polystyrenesulfonate | 25 | 25 | 25 | 25 |
| Carboxymethyl-cellulose sodium | 0.6 | 0.6 | 0.6 | 0.6 |
| Sorbitol | 8.0 | 8.0 | 8.0 | 8.0 |
| Saccharin sodium | 0.02 | 0.04 | 0.04 | 0.04 |
| Citric acid | - | 0.02 | 0.02 | 0.02 |
| Sodium citrate | - | 0.2 | 0.2 | 0.2 |
| Medium-chain fatty acid triglyceride | 6.0 | 4.0 | 6.0 | 7.0 |

(continued)

|  | No.67 | No.68 | No.69 | No.70 |
|---|---|---|---|---|
| Purified water | 25 | 25 | 25 | 25 |
| Total | 64.8 g | 62.86 g | 64.86 g | 65.86 g |
| Shape retaining property | ○ | ○ | ○ | ○ |
| Impression on ingestion | ○ | ○ | ○ | ○ |
| Water absorption capacity | ○ | ○ | ○ | ○ |
| [Note]<br>○: good, ×: poor, Δ: equivocal. | | | | |

(3) Evaluation test for thermal drying treatment of polystyrenesulfonate preparation

To determine whether or not similar results are obtained in the case of polystyrenesulfonate, an evaluation and investigation for thermal drying treatment of polystyrenesulfonate preparation were performed. With the composition (gram number) shown for sample No.68, reagents were kneaded, and thermal drying treatment by one-face heating or both-face heating was performed at drying temperature settings of normal temperature, 60˚C, 80˚C, 100˚C, 120˚C, 150˚C, 170˚C, and 200˚C in the same manner as Example 1. Regarding drying endpoint checks, the water activity was determined, and the endpoint was determined at the time when the water activity became not more than 0.55. Water activity was determined using an AW measuring system manufactured by Rotronic AG Company (AW-Palm). The results, along with void ratios, are shown in Table 29 below.

[Table 29]

| Experiment No. | Heat treatment temperature | Void ratio | Eating quality |
|---|---|---|---|
| 1 | Normal temperature/ one face | 21.3% | × |
| 2 | 60˚C/one face | 23.5% | × |
| 3 | 80˚C/one face | 23.6% | × |
| 4 | 100˚C/one face | 24.4% | × |
| 5 | 120˚C/one face | 27.6% | Δ |
| 6 | 120˚C/both faces | 30.1% | ○ |
| 7 | 150˚C/both faces | 33.4% | ○ |
| 8 | 170˚C/both faces | 29.4% | ○ |
| 9 | 200˚C/both faces | 30.3% | ○ |
| [Note]<br>○: good, ×: poor, Δ: equivocal. | | | |

It was found that the void ratio of the dry preparation of the present invention, as in the case of Example 1, is largely affected by heating temperature. It was also found that the void ratio is slightly influenced by one-face heating and both-face heating with a hot plate. From the above-described results from the evaluations of void ratio and eating quality, it was found that as in Example 1 there is a positive correlation between void ratio and impression on ingestion, and that a preparation with a good impression on ingestion is obtained if the void ratio is not less than 25%. It was also found that if the void ratio is not less than 30%, a preparation with a better impression on ingestion is obtained. Because microbial growth is reportedly impossible when the water activity is not more than 0.55, the preparation of the present invention has excellent stability to microorganisms during storage.

Test Example 1: Determination of the amount of water contained in preparation

General Test (loss on drying test) specified in the Japanese Pharmacopoeia

Sample No.70 was milled, 1.0 g was weighed out, and tested as directed in the loss on drying test for "Calcium Polystyrenesulfonate" (80°C, reduced pressure, 5 hours) specified in the Japanese Pharmacopoeia. As a result, the amount of water contained in sample No.70 was 3.5 w/w% of the preparation mass.

Test Example 2: Measurement of void ratio

Sample No.70 was weighed out and impregnated in a medium-chain fatty acid triglyceride at about 25°C, and allowed to stand under reduced pressure (0.09 MPa) for 5 minutes. After impregnation, the surplus triglyceride adhering to the sample was wiped off, and the sample mass was weighed. The void ratio was calculated using the following calculation formula: "Void ratio" (%) = [(mass after immersion - mass before immersion) ÷ (0.943) × 100] ÷ (mass after immersion) The results are shown in Table 30 below. Hence, it was shown that the above-described method of calculating the void ratio is highly reproducible, and can be used as an indicator for the preparation of the present invention.
[Table 30]

| Experiment No. | Mass before immersion | Mass after immersion | Void volume | Void ratio |
|---|---|---|---|---|
| 1 | 0.508 g | 0.741 g | 0.247 ml | 33.3% |
| 2 | 0.525 g | x0.766 g | 0.256 ml | 33.4% |
| 3 | 0.636 g | 0.929 g | 0.311 ml | 33.4% |

Using this method, the void ratio of a commercially available gel-like preparation of calcium polystyrenesulfonate salt (trade name: Argamate jelly, Sanwa Kagaku, EH04AK) was determined. The mass became minus after impregnation with the medium-chain fatty acid triglyceride; this is attributable to the evaporation of the water in the jelly preparation under reduced pressure. The results are shown in Table 31 below. From this result, it was shown that the void ratio of the commercially available gel-like preparation of calcium polystyrenesulfonate salt was zero.
[Table 31]

| Experiment No. | Mass before immersion | Mass after immersion | Void volume | Void ratio |
|---|---|---|---|---|
| 4 | 1.612 g | 1.555 g | -0.057 ml | -3.7% |
| 5 | 0.987 g | 0.960 g | -0.027 ml | -2.8% |
| 6 | 1.017 g | 0.985 g | -0.032 ml | -3.2% |

(4) Investigation of choice and effects of polymeric thickener

The effects of polymeric thickeners other than CMCNa were investigated. With sample No.70 as the reference, the reagents (numbers of grams) listed in Table 32-1 below were weighed out, and porous dry matrix preparations were prepared in the same manner as Example 2.
[Table 32-1]

| | No.70 | No.71 | No.72 | No.73 | No.74 | No.75 |
|---|---|---|---|---|---|---|
| Calcium polystyrenesulfonate | 25.0 | 25.0 | 25.0 | 25.0 | 25.0 | 25.0 |
| Polymeric thickener or gelling agent | CMCNa 0.6 | Guar gum 0.4 | Carrageenan 0.3 | Carrageenan 0.3 Carob bean gum 0.02 | Gelling agent/ agar 0.3 | Gelling agent/ glucomannan0.4 |

(continued)

|  | No.70 | No.71 | No.72 | No.73 | No.74 | No.75 |
|---|---|---|---|---|---|---|
| Sorbitol | 8.0 | 8.0 | 8.0 | 8.0 | 8.0 | 8.0 |
| Saccharin sodium | 0.04 | 0.04 | 0.04 | 0.04 | 0.04 | 0.04 |
| Citric acid | 0.02 | 0.02 | 0.02 | 0.02 | 0.02 | 0.02 |
| Sodium citrate | 0.2 | 0.2 | 0.2 | 0.2 | 0.2 | 0.2 |
| Medium-chain fatty acid triglyceride | 7.0 | 7.0 | 7.0 | 7.0 | 7.0 | 7.0 |
| Purified water | 25.0 | 25.0 | 25.0 | 25.0 | 25.0 | 25.0 |
| Void ratio | 33.4% | 30.6% | 15.3% | 28.5% | Immeasurable | Immeasurable |
| Shape retaining property | ○ | ○ | ○ | ○ | × | × |
| Impression on ingestion | ○ | ○ | × | ○ | × | × |
| [Note] ○: good, ×: poor, Δ: equivocal. | | | | | | |

From this result, it is clear that when a gelling agent that produces a brittle and easily collapsing gel, such as agar or glucomannan, is used, a mixed composition being prepared becomes brittle and easily collapsing, so that molding becomes difficult. When a reagent that produces a viscous solution was used as the polymeric thickener, a good porous dry matrix preparation could be obtained in, for example, a system with guar gum, or a system with carrageenan and carob bean gum.

Furthermore, the system was simplified, and the gelling agent (agar) and synthetic polymeric thickener (polyvinyl alcohol) were investigated again. The reagents (numbers of grams) listed in Table 32-2 below were weighed out, and porous dry matrix preparations were prepared in the same manner as Example 2.

[Table 32-2]

|  | 75-1 | 75-2 |
|---|---|---|
| Calcium polystyrenesulfonate | 25 | 25 |
| Polymeric thickener or gelling agent | Gelling agent/agar 0.5 | 30% polyvinyl alcohol: 5 |
| Powdered reduced maltose syrup | 5 | 5 |
| Medium-chain fatty acid | 3 | 3 |
| Purified water | 25 | 25 |
| (Moldability) / base material nature | Δ Easily cracking | × Not moldable |
| 150°C, 5 minutes |  |  |
| (Shape retaining property) | × (Collapses under small force) | × |
| (Eating quality) | Δ Wafer-like | Unevaluable |
| [Note] ○: good, ×: poor, Δ: equivocal. | | |

From this result, it was shown that even when agar was increased, shape retaining property did not improve, and the preparation is highly labile to collapse upon contact with water. In the case of polyvinyl alcohol, which is a synthetic polymeric thickener, the preparation became a whipped cream state and could not be molded. When the preparation was thermally dried with a hot plate while in a whipped cream state, water evaporated off instantaneously and a powder was formed. For this reason, eating quality could not be evaluated.

Furthermore, as polymeric thickeners, hydroxypropylcellulose as a water-soluble derivative of cellulose, acrylic acid starch as a starch derivative, and carboxyvinyl polymer as a synthetic polymeric thickener were investigated. The reagents (numbers of grams) listed in Table 32-3 below were weighed out, and porous dry matrix preparations were prepared in the same manner as Example 2.

[Table 32-3]

| | 75-3 | 75-4 | 75-5 |
|---|---|---|---|
| Calcium polystyrene-sulfonate | 25.0 | 25.0 | 25.0 |
| Polymeric thickener or gelling agent | Hydroxypropyl-cellulose 5.0 | Acrylic acid starch 3.5 | Carboxyvinyl polymer 1.0 |
| Sorbitol | 8.0 | 8.0 | 8.0 |
| Saccharin sodium | 0.04 | 0.04 | 0.04 |
| Citric acid | 0.02 | 0.02 | 0.02 |
| Sodium citrate | 0.2 | 0.2 | 0.2 |
| Medium-chain fatty acid triglyceride | 7.0 | 7.0 | 7.0 |
| Purified water | 25.0 | 25.0 | 25.0 |
| Void ratio | 32.4% | 42.6% | 34.3% |
| Shape retaining property | ○ | ○ | ○ |
| Impression on ingestion | ○ | ○ | ○ |
| Water absorption capacity | ○ | ○ | ○ |
| Shape retaining property after water absorption | × | × | ○ |
| [Note] ○: good, ×: poor, Δ: equivocal. | | | |

From this result, it was found that hydroxypropylcellulose, acrylic acid starch, and carboxyvinyl polymer can be used as the polymeric thickener in the present invention. When hydroxypropylcellulose or acrylic acid starch was used as the polymeric thickener, the preparation becomes so soft that the shape can no longer be maintained when the preparation is re-impregnated with water. However, it was found that this level is sufficient for a porous dry matrix preparation to be taken.

(5) Investigation of sweetener for improving impression on ingestion

An investigation was performed to determine whether or not the same results as Example 10 would be obtained if the excipient ingredient was replaced with polystyrenesulfonate. Changing the choice of sugar alcohol or sugar as the sweetener added, the ingredients (numbers of grams) listed in Table 33 below were weighed out, and dry preparations were prepared in the same manner as Example 2. The void ratio, as well as impression on ingestion, of the dry preparation obtained were evaluated. The results are shown in Table 33.

[Table 33]

| | No.70 | No.76 | No.77 | No.78 |
|---|---|---|---|---|
| Calcium polystyrene-sulfonate | 25 | 25 | 25 | 25 |
| Carboxymethyl-cellulose sodium | 0.6 | 0.6 | 0.6 | 0.6 |
| Sweetener | Sorbitol 8.0 | Mannitol 8.0 | Powdered reduced maltose syrup 8.0 | White sugar 8.0 |

(continued)

|  | No.70 | No.76 | No.77 | No.78 |
|---|---|---|---|---|
| Saccharin sodium | 0.04 | 0.04 | 0.04 | 0.04 |
| Citric acid | 0.02 | 0.02 | 0.02 | 0.02 |
| Sodium citrate | 0.2 | 0.2 | 0.2 | 0.2 |
| Medium-chain fatty acid triglyceride | 7.0 | 7.0 | 7.0 | 7.0 |
| Purified water | 25 | 25 | 25 | 25 |
| Total | 65.86 g | 65.86 g | 65.86 g | 65.86 g |
| Amount of water contained before drying (%) | 38.0 | 38.0 | 38.0 | 38.0 |
| Preparation void ratio (%) | 27.0 | 27.7 | 29.1 | 28.9 |
| Shape retaining property | ○ | ○ | ○ | ○ |
| Impression on ingestion | ○ | ○ | ○ | ○ |
| Water absorption capacity | ○ | ○ | ○ | ○ |
| [Note]<br>○: good, ×: poor, Δ: equivocal. | | | | |

It was found that even when polystyrenesulfonate was used as the excipient ingredient, as with the results obtained so far, the void ratio is not significantly influenced by the choice of sugar alcohol and sugar. It was also found that a dry preparation is obtained having a good shape retaining property and impression on ingestion.

(6) Investigation of the masking effect of edible oil/fat on the discomfort of polystyrenesulfonate

To mask the discomfort of polystyrenesulfonate, a medium-chain fatty acid triglyceride can be used as the edible oil/fat; the effects of the amount of triglyceride added were investigated. The ingredients (numbers of grams) listed in Table 34 below were weighed out, and dry preparations were prepared in the same manner as Example 2. The void ratio, as well as impression on ingestion, of the dry preparation obtained were evaluated. The results are shown in Table 34.

[Table 34]

|  | No.79 | No.80 | No.81 | No.82 | No.83 | No.84 |
|---|---|---|---|---|---|---|
| Calcium polystyrene-sulfonate | 25.0 | 25.0 | 25.0 | 25.0 | 25.0 | 25.0 |
| Carboxymethyl-cellulose sodium | 0.6 | 0.6 | 0.6 | 0.6 | 0.6 | 0.6 |
| Sorbitol | 8.0 | 8.0 | 8.0 | 8.0 | 8.0 | 8.0 |
| Saccharin sodium | 0.04 | 0.04 | 0.04 | 0.04 | 0.04 | 0.04 |
| Citric acid | 0.02 | 0.02 | 0.02 | 0.02 | 0.02 | 0.02 |
| Sodium citrate | 0.2 | 0.2 | 0.2 | 0.2 | 0.2 | 0.2 |
| Medium-chain fatty acid triglyceride | None | 3 | 4 | 6 | 7 | 8 |
| Purified water | 25.0 | 25.0 | 25.0 | 25.0 | 25.0 | 25.0 |
| Amount of water contained before drying (%) | 42.5 | 40.4 | 39.8 | 38.5 | 38.0 | 37.4 |
| Preparation void ratio (%) | 37.4 | 33.3 | 33.4 | 30.2 | 27.0 | 27.1 |

(continued)

|  | No.79 | No.80 | No.81 | No.82 | No.83 | No.84 |
|---|---|---|---|---|---|---|
| Impression on ingestion | × | ○ | ○ | ○ | ○ | ○ |
| [Note]<br>○: good, ×: poor, Δ: equivocal. | | | | | | |

In the dry preparation not containing the medium-chain fatty acid triglyceride of sample No.79, the void ratio was high at 37%, and the tooth touch was excellent, but in terms of eating quality and impression on ingestion, the tongue touch was bad, and the discomfort of polystyrenesulfonate was intensely felt.

From this result, it was demonstrated that if at least not less than 0.12 parts by mass of a medium-chain fatty acid triglyceride is present per part by mass of the polystyrenesulfonate, the discomfort and rough feeling of polystyrenesulfonate can be masked.

When this result was evaluated by pore maintenance rate, the results shown in Table 35 below are obtained.

[Table 35]

|  | No.79 | No.80 | No.81 | No.82 | No.83 | No.84 |
|---|---|---|---|---|---|---|
| Pore volume maintenance rate (%) | 88.0 | 82.4 | 83.9 | 78.4 | 71.1 | 72.5 |
| Triglyceride content (%) in dry preparation | 0 | 8.1 | 10.6 | 15.1 | 17.1 | 19.1 |
| Triglycerides/ excipient ingredient | 0 | 0.12 | 0.16 | 0.23 | 0.27 | 0.31 |

As shown in Example 6, it was shown that even when polystyrenesulfonate was used as the excipient ingredient, in the absence of a medium-chain fatty acid triglyceride, the void ratio and pore volume maintenance rate were the highest. This finding shows that to cover the surface of the polystyrenesulfonate that forms the porous dry matrix to mask discomfort, if the content in the dry preparation is not less than about 8%, the discomfort and rough feeling of polystyrenesulfonate can be masked. As the triglyceride content increased, the pore volume maintenance rate decreased gradually. From this finding, it is thought that the edible oil/fat covers the surface of the polystyrenesulfonate that forms the pores, and also fills the voids in the matrix.

(7) Investigation of masking effect by choice of edible oil/fat

As shown in Example 7, with polystyrenesulfonate as the excipient ingredient, when the choice of edible oil/fat was replaced with one other than a medium-chain fatty acid triglyceride, whether or not the same masking effect was obtained was determined. The ingredients (numbers of grams) listed in Table 36 below were weighed out, and dry preparations were prepared in the same manner as Example 2. The impression on ingestion of the dry preparation obtained was evaluated, and the results are shown in Table 36. This result shows that even when the choice of edible oil/fat is changed, the discomfort masking effect is not significantly influenced, although there is some influence of flavor, and this agrees with the results in Example 7.

[Table 36]

|  | No.85 | No. 86 | No. 87 | No. 89 | No. 90 | No. 91 |
|---|---|---|---|---|---|---|
| Calcium polystyrene-sulfonate | 25.0 | 25.0 | 25.0 | 25.0 | 25.0 | 25.0 |
| Carboxymethyl-cellulose sodium | 0.6 | 0.6 | 0.6 | 0.6 | 0.6 | 0.6 |
| D-sorbitol | 8.0 | 8.0 | 8.0 | 8.0 | 8.0 | 8.0 |
| Saccharin sodium | 0.04 | 0.04 | 0.04 | 0.04 | 0.04 | 0.04 |

(continued)

|  | No.85 | No. 86 | No. 87 | No. 89 | No. 90 | No. 91 |
|---|---|---|---|---|---|---|
| Citric acid | 0.02 | 0.02 | 0.02 | 0.02 | 0.02 | 0.02 |
| Sodium citrate | 0.2 | 0.2 | 0.2 | 0.2 | 0.2 | 0.2 |
| Edible oil/fat | Medium-chain fatty acid triglycerides 7.0 | Salad oil 7.0 | Cottonseed oil 7.0 | Canola oil 7.0 | Corn oil 7.0 | Safflower oil 7.0 |
| Purified water | 25.0 | 25.0 | 25.0 | 25.0 | 25.0 | 25.0 |
| Total | 65.86 | 65.86 | 65.86 | 65.86 | 65.86 | 65.86 |
| Impression on ingestion | ○ | ○ | ○ | ○ | ○ | ○ |
| [Note]<br>○: good, ×: poor, △: equivocal. | | | | | | |

(8) Effects of changes in polystyrenesulfonate particle diameter on eating quality

In the porous dry matrix preparation of the present invention, to evaluate the effects of the particle diameter of polystyrenesulfonate on impression on ingestion, using the following samples, a dry preparation was prepared with the same composition as sample No.85 in the same manner as Example 2.
Particle diameter of calcium polyethylenesulfonate:

<1> 24.9 to 81.9 (median diameter 48.85) $\mu$m,
<2> 28.7 to 101.2 (median diameter 59.9) $\mu$m,
<3> 30.5 to 128.9 (median diameter 70.96) $\mu$m,

Five panelists took dry preparations prepared using calcium polystyrenesulfonates having the foregoing three particle diameters, and the effects of particle diameter on the impression on ingestion were examined. The results are summarized in Table 37 below.

No discomfort: ○
Slight discomfort: △
Discomfort and rough feeling: ×

[Table 37]

| Particle diameter ($\mu$m) | ○ | △ | × | Sensory evaluation test |
|---|---|---|---|---|
| 24.9 to 81.9 | 5 panelists | 0 panelist | 0 panelist | ○ |
| 28.7 to 101.2 | 5 panelists | 0 panelist | 0 panelist | ○ |
| 30.5 to 128.9 | 5 panelists | 0 panelist | 0 panelist | ○ |
| [Note]<br>○: good, ×: poor, △: equivocal. | | | | |

As shown above, it was demonstrated that the preparation of the present invention differs significantly from a commonly known gel-like preparation, and its impression on ingestion is not so influenced by the particle diameter of polystyrenesulfonate.
Therefore, in the preparation of the present invention, despite the change in the particle diameter of polystyrenesulfonate, stable impression on ingestion with constant composition was obtained, and additionally, the rough feeling characteristic of polystyrenesulfonate was well masked by the edible oil/fat; the preparation has excellent eating quality. Preferably, the particle diameter is suitably as small as possible for the sake of preparation making; specifically, one having a particle diameter of 5 to 130 μm is preferable.

(9) Evaluation of void ratio and water absorption capacity of preparation, and impression on ingestion of hydrated matrix preparation

Using samples prepared in (3) above (Experiment Nos. 2 to 9), impregnated with purified water, the relationship between the void ratio and water absorption capacity of the preparation of the present invention was evaluated. The water absorption time endpoint was determined on the basis of the presence or absence of a feeling of core when the sample was held up.
Regarding water absorption capacity, about 1 g of the above-described sample was weighed out, and the sample was immersed in about 40 ml of purified water contained in a Petri dish, 9 cm in diameter, at room temperature. The sample was allowed to stand for a specified time, after which the sample was removed, pinched by fingertips, and examined for a core.
The results are shown in Table 38 below.
[Table 38]

| Experiment No. | Heat treatment temperature | Void ratio | Water absorption capacity | Impression on ingestion after reimpregnation with water |
|---|---|---|---|---|
| 2 | 60˚C/one face | 23.5% | Does not absorb water. | - |
| 3 | 80˚C/one face | 23.6% | Does not absorb water. | - |
| 4 | 100˚C/one face | 24.4% | 45 minutes | ○ |
| 5 | 120˚C/one face | 27.6% | 4 minutes | ○ |
| 6 | 120˚C/both faces | 30.1% | 1 minute | ○ |
| 7 | 150˚C/both faces | 33.4% | 1 minute | ○ |
| 8 | 170˚C/both faces | 29.4% | 1 minute | ○ |
| 9 | 200˚C/both faces | 30.3% | 1 minute | ○ |

[Note]
Sensory evaluation test results were rated with ○ given when not less than 60% of the panelists reported the absence of discomfort. When not less than 60% of the panelists felt discomfort, x was given. An intermediate rating is shown by △. - indicates that water absorption capacity is insufficient to permit an evaluation.

From this result, it was found that even with the same composition of preparation, a difference in heat treatment temperature results in a difference in void ratio; furthermore, water absorption capacity (water absorption time) is significantly influenced by this difference in void ratio. All wet water-carrying preparations after completion of water absorption offered a good impression on ingestion. Particularly, it was shown that if the dry preparation preferably has a void ratio of not less than 29%, water absorption time tends to be within 1 minute.
The water-carrying preparation of the present invention retained a material strength such that it can be pinched with fingers without collapsing under a force being exerted in a state wherein water had permeated sufficiently in the preparation. This water-carrying preparation is a preparation having a touch like a fine sponge being immersed in water, and releases the water being carried thereby when pinched. Hence, the water-carrying preparation of the present invention is not an ordinary jelly-like preparation, but can, for example, be described as a water-containing sponge-like preparation.
In the case of a commercially available jelly of calcium polystyrenesulfonate salt (trade name: Argamate jelly, Sanwa Kagaku, EH04AK), exerting a force resulted in only collapse of the jelly and no water separation was observed. Meanwhile, the water-carrying preparation of the present invention does not collapse even when a force is exerted thereon, with water being separated as if squeezed out. As shown in these results, the preparation of the present invention did not turn to an ordinary jelly shape even when re-impregnated with water.

(10) Change in additive composition for calorific reductions and improvement of impression on ingestion

It is likely that patients to receive the preparation of the present invention include patients undergoing renal dialysis; special attention is necessary concerning calorie intake, as well as water intake, for this patient population. In the preparation of the present invention, it is desirable that a edible oil/fat and a sweetener be formulated to mask the rough feeling of polystyrenesulfonate to improve eating quality; however, because these additives have high calorific values, a reduction in the amount added is demanded. Hence, to investigate the possibility of a reduction in the amount added and a reduction in calorific level, as the edible oil/fat, replacement with concentrated glycerin, which has a less calorific value, was investigated. Hence, the ingredients (numbers of grams) listed in Table 39 below were weighed out, and preparations were prepared in the same manner as Example 2. Both the impression on ingestion and water absorption capacity of the dry preparation obtained were evaluated.

[Table 39]

| | No.77 | No.92 | No.93 | No.94 | No.95 | No.96 |
|---|---|---|---|---|---|---|
| Calcium polystyrene-sulfonate | 25.0 | 25.0 | 25.0 | 25.0 | 25.0 | 25.0 |
| Carboxymethyl-cellulose sodium | 0.6 | 0.6 | 0.6 | 0.6 | 0.6 | 0.6 |
| Saccharin sodium | 0.04 | 0.02 | 0.02 | 0.02 | 0.02 | 0.02 |
| Citric acid | 0.02 | 0.02 | 0.02 | 0.02 | 0.02 | 0.02 |
| Sodium citrate | 0.2 | 0.2 | 0.2 | 0.2 | 0.2 | 0.2 |
| Powdered reduced maltose syrup | 8.0 | 6.0 | 6.0 | 4.0 | 3.0 | 3.0 |
| Medium-chain fatty acid triglyceride | 7.0 | 4.0 | 4.0 | 3.3 | 3.3 | 1.5 |
| Purified water | 25.0 | 25.0 | 25.0 | 26.0 | 26.0 | 27.0 |
| Concentrated glycerin | | | 2.0 | 6.0 | 6.0 | 6.0 |
| Cornstarch | | | | | | 0.75 |
| Impression on ingestion | ○ | ○ | ○ | ○ | ○ | ○ |
| Water absorption capacity (water absorption time) | ○ 1 min | Δ 2 min | Δ 3 min | ○ 1.5 min | ○ 1 min | Δ 6 min |
| [Note]<br>○: good, ×: poor, Δ: equivocal. | | | | | | |

As shown by this result, by adding concentrated glycerin as the edible oil/fat, the amount of powdered reduced maltose syrup added and the amount of medium-chain fatty acid triglyceride added were successfully reduced while maintaining a good impression on ingestion. Meanwhile, when a starch such as cornstarch was added, the impression on ingestion improved, but the water absorption capacity of the preparation tended to decrease.

(11) Changing the amount of CMCNa added to improve impression on ingestion and water absorption capacity

As described in (10) above, when the amounts of powdered reduced maltose syrup and medium-chain fatty acid triglyceride were reduced to decrease the calorific level of the preparation of the present invention, the impression on ingestion tended to worsen slightly compared with the composition before the reduction, and the water absorption capacity also tended to worsen. Hence, the association of changes in the amount of CMCNa added and impression on ingestion, water absorption capacity was investigated. The reagents (numbers of grams) listed in Table 40 below were weighed out, and preparations were prepared in the same manner as Example 2. In molding before drying, each preparation was molded to obtain a thickness of about 4 mm and a length-width size of about 3.0 cm x about 9.0 cm. The preparation obtained maintained a thickness of about 4 mm. The impression on ingestion, as well as the water absorption capacity of the dry preparation obtained, were evaluated.

[Table 40]

| | No.97 | No.98 | No.99 | No.100 |
|---|---|---|---|---|
| Calcium polystyrenesulfonate | 25.0 | 25.0 | 25.0 | 25.0 |

(continued)

| | No.97 | No.98 | No.99 | No.100 |
|---|---|---|---|---|
| Carboxymethyl-cellulose sodium | 0.6 | 0.5 | 0.4 | 0.3 |
| Saccharin sodium | 0.02 | 0.02 | 0.02 | 0.02 |
| Citric acid | 0.02 | 0.02 | 0.02 | 0.02 |
| Sodium citrate | 0.2 | 0.2 | 0.2 | 0.2 |
| Powdered reduced maltose syrup | 4.0 | 4.0 | 4.0 | 4.0 |
| Medium-chain fatty acid triglyceride | 1.5 | 1.5 | 1.5 | 1.5 |
| Purified water | 25.0 | 25.0 | 25.0 | 26.0 |
| Concentrated glycerin | 1.6 | 1.6 | 1.6 | 1.6 |
| Glycerin | 5.0 | 5.0 | 5.0 | 5.0 |
| Cornstarch | 0.5 | 0.5 | 0.5 | 0.5 |
| Void ratio (%) | 39.4 | 41.8 | 41.5 | 44.8 |
| Shape retaining property | ○ | ○ | Δ Surface cracks observed. Expanded about 10% laterally. | × Surface cracks observed. Expanded considerably laterally. |
| Impression on ingestion | ○ | ○ | ○ | ○ |
| Water absorption capacity (water absorption time) | ○ 5 min | ○ 1 min | ○ 1 min | ○ 45 sec |
| Shape retaining property after water absorption | ○ | ○ | ○ | Δ Slightly brittle |
| Impression on ingestion after water absorption | ○ | ○ | ○ | ○ |
| [Note] ○: good, ×: poor, Δ: equivocal. | | | | |

The water absorption capacity of sample No.99 and No.100 was better than that of sample No.97 and No.98; this result indicates better water absorption capacity for the dry matrix preparations of sample No.99 and No.100 because they expanded and they had many cracks on their surfaces.
Furthermore, when this result was evaluated by pore volume maintenance rate, the results shown in Table 41 below were obtained.
[Table 41]

| | No.97 | No.98 | No.99 | No.100 |
|---|---|---|---|---|
| Amount of water contained (%) | 39.4 | 39.5 | 39.5 | 39.6 |
| Void ratio (%) | 39.4 | 41.8 | 41.5 | 44.8 |
| Pore volume maintenance rate (%) | 100.0 | 105.8 | 105.1 | 113.1 |
| CMCNa content in dry preparation | 1.6% | 1.3% | 1.0% | 0.8% |

(continued)

| | No.97 | No.98 | No.99 | No.100 |
|---|---|---|---|---|
| Calcium polystyrenesulfonate/ CMCNa | 41.7 | 50.0 | 62.5 | 83.3 |
| Calcium polystyrenesulfonate content in dry preparation | 65.0% | 65.2% | 65.4% | 65.5% |
| Content of excipient ingredient and CMCNa before drying | 40.4% | 40.3% | 40.2% | 40.1% |
| Shape retaining property | ○ | ○ | △ Surface cracks observed. Expanded about 10% laterally. | × Surface cracks observed. Expanded considerably laterally. |
| Impression on ingestion | ○ | ○ | ○ | ○ |
| Water absorption capacity (water absorption time) | ○ 5 min | ○ 2.5 min | ○ 1 min | ○ 45 sec |

From this result, it is found that when the content of excipient ingredient was high, and the amount of CMCNa as the structure ingredient was small, the matrix structure does not endure the pressure of vaporization of water during drying, and pores burst, resulting in size expansion at the time of drying compared with the size at the time of molding. As a result, the pore volume maintenance rate exceeds 100%, as seen in sample No.99 and sample No.100, and many cracks are formed on the preparation surfaces.

From these results, taking shape retaining property into consideration, it is found that the CMCNa content (in dry preparation) is preferably not less than about 1%. It was also found that the ratio by mass of the excipient ingredient and CMCNa is preferably not more than about 63.

(12) Investigation of improvement of water absorption capacity of preparation

To further improve the water absorption capacity of the preparations described in (10) and (11) above, the effects of addition of emulsifier were investigated. Focusing on the composition of sample No.98, the reagents (numbers of grams) listed in Table 42 below were weighed out, and preparations were prepared in the same manner as Example 2. As in (11) above, in molding before drying, the preparation was molded to have a thickness of about 4 mm and a length-width size of about 3.0 cm x about 9.0 cm. The preparation obtained maintained a thickness of about 4 mm. The eating quality and impression on ingestion, as well as the water absorption capacity of this dry preparation, were evaluated.

[Table 42]

| | No.98 | No.101 | No.102 | No.103 |
|---|---|---|---|---|
| Calcium polystyrenesulfonate | 25.0 | 25.0 | 25.0 | 25.0 |
| Carboxymethyl-cellulose sodium | 0.5 | 0.5 | 0.5 | 0.5 |
| Saccharin sodium | 0.02 | 0.02 | 0.02 | 0.02 |
| Citric acid | 0.02 | 0.02 | 0.02 | 0.02 |
| Sodium citrate | 0.2 | 0.2 | 0.2 | 0.2 |
| Powdered reduced maltose syrup | 4.0 | 4.0 | 4.0 | 4.0 |
| Medium-chain fatty acid triglyceride | 1.5 | 1.5 | 1.5 | 1.5 |

(continued)

| | No.98 | No.101 | No.102 | No.103 |
|---|---|---|---|---|
| Purified water | 25.0 | 25.0 | 25.0 | 25.0 |
| Concentrated glycerin | 1.6 | 1.6 | 1.6 | 1.6 |
| Glycerin | 5.0 | 5.0 | 5.0 | 5.0 |
| Cornstarch | 0.5 | 0.5 | 0.75 | 0.75 |
| Emulsifier | | Polyvinyl alcohol 0.003 | Polyvinyl alcohol 0.03 | Sucrose fatty acid ester 0.05 |
| Void ratio | 41.8% | 39.5% | 42.9% | 38.0% |
| Impression on ingestion | ○ | ○ | ○ | ○ |
| Water absorption capacity (water absorption time) | ○ 2.5 min | ○ 1.5 min | ○ 1 min | ○ 2 min |
| [Note]<br>○: good, ×: poor, Δ: equivocal. | | | | |

As shown by the above-described results, it was found that by the addition of an emulsifier, water absorption capacity can be improved. Taking the above-described data and the results of investigations thus far into consideration, it can be concluded that water absorption capacity is affected by CMCNa, cornstarch, powdered reduced maltose syrup, emulsifier and the like, and that additives other than emulsifiers tend to reduce water absorption capacity when added at increased amounts.
When this result was evaluated by pore volume maintenance rate, the results shown in Table 43 below were obtained. As shown by this result, it was found that when an emulsifier is used, the pore volume maintenance rate does not change significantly but water absorption capacity improves.
[Table 43]

| | No.98 | No.101 | No.102 | No.103 |
|---|---|---|---|---|
| Amount hydrated (%) | 39.5 | 39.5 | 39.3 | 39.3 |
| Void ratio | 41.8% | 39.5% | 42.9% | 38.0% |
| Pore volume maintenance rate | 105.8% | 100.0% | 109.2 | 96.7% |
| Impression on ingestion | ○ | ○ | ○ | ○ |
| Water absorption capacity (water absorption time) | ○ 2.5 minutes | ○ 1.5 minutes | ○ 1 minute | ○ 2 minutes |

(13) Potassium exchange capacity test of preparation

Regarding a commercially available jelly of calcium polystyrenesulfonate salt (trade name: Argamate jelly, Sanwa Kagaku, EH04AK), non-patent document 1 describes, "the amount of potassium removed by PS-Ca in the jelly-like preparation over time depended on the degree of division of the jelly-like preparation. The undivided PS-Ca jelly-like preparation little collapsed in the dissolution tester, and was incapable of exchanging with potassium. When the preparation was divided into 8 portions, an increase in potassium exchange speed was observed, but 2 hours later the exchange rate nearly reached a peak, and was up to about 60% relative to the PS-Ca powder." (line 2 from bottom on page 266 to line 3 on page 267).
Hence, regarding the preparation of the present invention, whether or not a change in potassium exchange rate occurs depending on the degree of division of the preparation was determined. For the preparation of the present invention, a potassium exchange rate evaluation test was performed in the same manner as the potassium exchange capacity test for calcium polystyrenesulfonate (C-4130) specified in the Japanese Pharmacopoeia. First, sample No.70 preparation while remaining in a tabular form, in an amount equivalent to 1 g of dry calcium polystyrenesulfonate, was weighed out in a stoppered glass container, 50 mL of potassium standard solution was added, and the mixture was shaken and stirred at room temperature for 120 minutes. After decantation, the solution was filtered, 10 mL of the filtrate was weighed out, after which 0.02 mol/L hydrochloric acid test solution was added to make 100 mL, and this solution was used as the sample solution. Using this reagent solution and potassium standard solution, the test was performed by ion chromatography, and the potassium exchange capacity per gram of calcium polystyrenesulfonate of sample No. 70 was calculated. Likewise, for Kalimate dry syrup (Kowa Pharmaceutical Co., Ltd.), a potassium exchange capacity test was performed, and potassium exchange capacity per gram of calcium polystyrenesulfonate was calculated. The results are shown in Table 44 below.

[Table 44]

|  | Potassium exchange capacity |
|---|---|
| Tabular sample of sample No. 70 | 55.1 mg |
| Kalimate dry syrup | 55.8 mg |

From this result, in the preparation of the present invention, even without division or milling, potassium exchange capacity remained almost unchanged compared with polystyrenesulfonate alone (Kalimate dry syrup (Kowa Pharmaceutical Co., Ltd.). It was demonstrated that in the preparation of the present invention, changes in preparation size and shape little affect the potassium exchange rate.

(14) Potassium exchange capacity comparative test between the preparation and a commercially available jelly of calcium polystyrenesulfonate salt

As the preparation of the present invention, sample No.67 was used, and as the commercially available jelly of calcium polystyrenesulfonate salt, Argamate jelly (Sanwa Kagaku Kenkyusho Co., Ltd., EH04AK) was used. Regarding the test method, a method according to the paddle method described in a reference document (Yakuzaigaku, 60(4), 261-270(2000)) was used.

About 8 g of sample No.70 and 25 g of the jelly were weighed out (corresponding to 5 g of calcium polystyrenesulfonate), and each, without being divided or disrupted, was added to a test liquid (prepared by adding 50 ml of 0.639 M potassium chloride solution to 850 ml of water), and the mixture was stirred. The paddle rotation rate was 100 rpm, and test liquid temperature was kept at about 37°C.

To measure the amount of potassium removed from the test liquid, the test liquid was sampled over time and filtered, after which each sampled aliquot was diluted with 0.02 N hydrochloric acid as appropriate, and tested by ion chromatography, and potassium exchange capacities of sample No.70 and the jelly per gram of calcium polystyrenesulfonate were calculated.

The results are shown in Table 45 below and FIG. 1.

[Table 45]

| Time (minutes) | Example 3 | Commercially available jelly |
|---|---|---|
| 0 | 0 | 0 |
| 30 | 13.15 | 3.12 |
| 60 | 17.18 | 0.17 |
| 120 | 27.67 | 4.37 |

From this result, it was shown that the dry preparation of the present invention is capable of quicker potassium exchange compared with the commercially available jelly.

Hence, it was demonstrated that compared with the commercially available jelly of polystyrenesulfonate described in the aforementioned reference document, the preparation of the present invention is little influenced by the degree of preparation division (preparation shape and size) in terms of potassium exchange rate (capacity).

Example 16: Investigation of porous dry matrix preparation of theophylline

A mixture of 0.15 g of theophylline, 1.0 g of carboxymethylcellulose Na, 5.0 g of crystalline cellulose, 1.0 g of a medium-chain fatty acid triglyceride, 4.0 g of white sugar, and 13.0 g of purified water was kneaded (the amount of water contained 53.8%), and a porous dry matrix preparation was prepared in the same manner as Example 2.

The porous dry matrix preparation obtained had a void ratio of 36.2% and offered a good impression on ingestion, and the bitterness had been mitigated. It was also shown that the preparation exhibited good shape retaining property even when impregnated with water, and offered a good impression on ingestion for a porous water-carrying matrix preparation.

Example 17: Investigation of porous dry matrix preparation containing Livact granules

4.0 g (1.144 g of valine, 1.904 g of leucine, 0.952 g of isoleucine) of Livact granules (Ajinomoto Pharma Co., Ltd.), 0.36 g of CMCNa, 3.2 g of crystalline cellulose, 4.0 g of powdered reduced maltose syrup, 3.6 g of a medium-chain fatty acid triglyceride, 0.6 g of sodium chloride, and 15.0 g of purified water were mixed, and a porous dry matrix preparation was prepared in the same manner as Example 2.

The porous dry matrix preparation obtained had slightly poor shape retaining property, but had mitigated bitterness and offered a good impression on ingestion. It was also shown that even when the preparation was impregnated with water, the shape retaining property was good, and the water absorption capacity was also good.

Example 18: Investigation of Kremezin-containing porous dry matrix preparation

(1) An investigational preparation of the following composition was investigated using crystalline cellulose as the excipient ingredient. 5.0 g of crystalline cellulose, 0.12 g of CMCNa, 0.2 g of starch, 1.2 g of powdered reduced maltose syrup, 1.0 g of a medium-chain fatty acid triglyceride, 0.2 g of concentrated glycerin, 0.04 g of Na citrate, 0.004 g of citric acid, 0.004 g of saccharin sodium, and 5.0 g of purified water were mixed, and a porous dry matrix preparation was prepared in the same manner as Example 2.

The porous dry matrix preparation obtained had a good shape retaining property, and offered a good impression on ingestion with a soft touch.

(2) Next, using Kremezin (Kureha Chemical Industry Co., Ltd.) as the excipient ingredient, preparation making with the following composition was investigated. 5.0 g of Kremezin, 0.12 g of CMCNa, 0.2 g of starch, 1.2 g of powdered reduced maltose syrup, 1.0 g of a medium-chain fatty acid triglyceride, 0.2 g of concentrated glycerin, 0.04 g of Na citrate, 0.004 g of citric acid, 0.004 g of saccharin sodium, and 5.0 g of purified water were mixed, and a porous dry matrix preparation was prepared in the same manner as Example 2.

Likewise, the porous dry matrix preparation obtained had a good shape retaining property and offered a good impression on ingestion. Even when the preparation was impregnated with water, the shape retaining property was good, and the water absorption capacity was also good.

Example 19: Production of preparations by drug impregnation

By preparing a porous dry matrix composition, impregnating the porous dry matrix composition with a solution containing each desired drug, and drying the composition, porous dry matrix preparations containing the following desired drugs were prepared.

(1) Preparation of porous dry matrix composition

A mixture of 1.0 g of carboxymethylcellulose Na, 5.0 g of crystalline cellulose, 3.0 g of a medium-chain fatty acid triglyceride, 4.0 g of white sugar, and 16.0 g of purified water was kneaded (the amount of water contained 55.2%), a porous dry matrix composition was prepared in the same manner as Example 2 (void ratio 30.6%).

(2) Porous dry matrix preparation containing metformin hydrochloride

31.25 mg of metformin hydrochloride was dissolved in 0.375 ml of purified water. The solution was added drop by drop to, and impregnated in, about 1 g of porous dry matrix composition. After impregnation, drying treatment was performed at 80˚C for 30 minutes under normal pressure to yield a desired metformin hydrochloride-containing porous dry matrix preparation. The water activity after drying was 0.518. Regarding the impression on ingestion of the dry preparation obtained, the unpleasant taste characteristic of metformin hydrochloride was absent; a preparation having a slightly salty taste was obtained.

(3) Ketoprofen-containing preparation

15.0 mg of ketoprofen was dissolved in 0.2 g of a medium-chain fatty acid triglyceride (heated at 80˚C for 10 minutes). This solution was impregnated in about 1 g of porous dry matrix composition. The impression on ingestion of the preparation obtained was good.

Test Example 3: Water activity evaluation test

Water activity was determined using an AW measuring system manufactured by Rotronic AG Company (AW-Palm). About 2 g of each of the samples of test Nos.1 to 7 in Example 1 was collected, and measurements were performed. The results are shown in Table 46 below.
[Table 46]

| Test No. | Thermal drying temperature | Water activity |
|---|---|---|
| 1 | 80˚C | 0.387 |
| 2 | 100˚C | 0.399 |
| 3 | 120˚C | 0.332 |
| 4 | 140˚C | 0.348 |
| 5 | 150˚C | 0.398 |
| 6 | 170˚C | 0.363 |
| 7 | 200˚C | 0.423 |

Test Example 4: Evaluation test for the amount of water contained in porous dry matrix preparation

Test No.5 in Example 1 was milled, about 1.0 g was weighed out, 10 ml of dehydrated methanol was added, and after tight sealing, ultrasonic stirring was performed for 30 minutes. The product was allowed to stand for 10 minutes, 2 ml of the supernatant was collected, and water was quantified by the Karl-Fischer method (manufactured by Kyoto Electronics, MKS-500). As a result, it was shown that the amount of water contained was not more than 10%.
This application is based on a patent application No. 2006-349401 filed in Japan on December 26, 2006, the contents of which are incorporated in full herein by this reference.

**Claims**

1. A porous dry matrix preparation comprising at least a polymeric thickener and an excipient ingredient, wherein

   (1) the content of the polymeric thickener is down to 0.5 w/w% and up to 14 w/w%,
   (2) the content of the excipient ingredient is down to 30 w/w% and up to 80 w/w%,
   (3) the void ratio is not less than 20%, and
   (4) the water activity is not more than 0.55, or water content is not more than 10 w/w%.

2. The preparation of claim 1, wherein the polymeric thickener comprises carboxymethylcellulose and/or a salt thereof.

3. The preparation of claim 2, wherein the carboxymethylcellulose salt is carboxymethylcellulose sodium or carboxymethylcellulose calcium.

4. The preparation of any of claims 1 to 3, wherein the excipient ingredient is a powder that is almost insoluble in water.

5. The preparation of claim 4, wherein the excipient ingredient being the powder that is almost insoluble in water is one kind or a mixture of two or more kinds selected from the group consisting of cellulose-series polymers, starch, synthetic polymers, and activated charcoal.

6. The preparation of claim 5, wherein the cellulose-series polymer is crystalline cellulose.

7. The preparation of any of claims 1 to 6, comprising an edible oil/fat.

8. The preparation of any of claims 1 to 7, comprising a sugar and/or a sugar alcohol.

9. The preparation of claim 8, wherein the sugar and/or the sugar alcohol is one kind or a mixture of two or more kinds selected from the group consisting of white sugar, powdered reduced maltose syrup, sorbitol, and mannitol.

10. The preparation of any of claims 1 to 9, comprising a pharmacologically effective ingredient.

11. A porous dry matrix preparation comprising at least polymeric thickener and a polystyrenesulfonate, wherein

(1) the content of the polymeric thickener is down to 0.5 w/w% and up to 14 w/w%,
(2) the content of the polystyrenesulfonate is down to 30 w/w% and up to 80 w/w%,
(3) the void ratio is not less than 20%, and
(4) the water activity is not more than 0.55, or water content is not more than 10 w/w%.

12. The preparation of claim 11, wherein the polymeric thickener comprises carboxymethylcellulose and/or a salt thereof.

13. The preparation of claim 12, wherein the carboxymethylcellulose salt is carboxymethylcellulose sodium or carboxymethylcellulose calcium.

14. The preparation of any of claims 11 to 13, wherein the polystyrenesulfonate is calcium polystyrenesulfonate or sodium polystyrenesulfonate.

15. The preparation of any of claims 11 to 14 above, comprising an edible oil/fat.

16. The preparation of any of claims 11 to 15, comprising a sugar and/or a sugar alcohol.

17. The preparation of claim 16, wherein the sugar and/or the sugar alcohol is one kind or a mixture of two or more kinds selected from the group consisting of white sugar, powdered reduced maltose syrup, sorbitol, and mannitol.

18. The preparation of any of claims 11 to 17, comprising a pharmacologically effective ingredient.

19. The preparation of claim 18, wherein the polystyrenesulfonate is also a pharmacologically effective ingredient.

20. A porous dry matrix preparation comprising at least carboxymethylcellulose sodium, calcium polystyrenesulfonate, a sugar and/or a sugar alcohol and an edible oil/fat, wherein

(1) the content of the carboxymethylcellulose sodium is down to 0.5 w/w% and up to 14 w/w%,
(2) the content of the calcium polystyrenesulfonate is down to 30 w/w% and up to 80 w/w%,
(3) the void ratio is not less than 20%, and
(4) the water activity is not more than 0.55, or water content is not more than 10 w/w%.

21. The preparation of claim 20, wherein the sugar and/or the sugar alcohol is one kind or a mixture of two or more kinds selected from the group consisting of white sugar, powdered reduced maltose syrup, sorbitol, and mannitol.

22. A porous dry matrix preparation comprising at least polymeric thickener and an excipient ingredient, wherein

(1) the content of the polymeric thickener is down to 0.5 w/w% and up to 14 w/w%,
(2) the content of the excipient ingredient is down to 30 w/w% and up to 80 w/w%,
(3) the void ratio is not less than 20%, and
(4) the water activity is not more than 0.55, or water content is not more than 10 w/w%,

and, furthermore, wherein the preparation completes water absorption within 15 minutes to become a hydrated composition that does not collapse when impregnated with water.

23. The preparation of claim 22, wherein the polymeric thickener comprises carboxymethylcellulose and/or a salt thereof.

24. The preparation of claim 23, wherein the carboxymethylcellulose salt is carboxymethylcellulose sodium or carboxymethylcellulose calcium.

25. The preparation of any of claims 22 to 24 above, wherein the excipient ingredient is a powder that is almost insoluble in water.

**26.** The preparation of claim 25, wherein the excipient ingredient being the powder that is almost insoluble in water is one kind or a mixture of two or more kinds selected from the group consisting of cellulose-series polymers, starch, synthetic polymers, and activated charcoal.

**27.** The preparation of claim 26, wherein the cellulose-series polymer is crystalline cellulose.

**28.** The preparation of any of claims 22 to 27, comprising an edible oil/fat.

**29.** The preparation of any of claims 22 to 28, comprising a sugar and/or a sugar alcohol.

**30.** The preparation of claim 29, wherein the sugar and/or the sugar alcohol is one kind or a mixture of two or more kinds selected from the group consisting of white sugar, powdered reduced maltose syrup, sorbitol, and mannitol.

**31.** The preparation of any of claims 22 to 30, comprising a pharmacologically effective ingredient.

**32.** A porous dry matrix preparation comprising at least a polymeric thickener and polystyrenesulfonate, wherein

(1) the content of the polymeric thickener is down to 0.5 w/w% and up to 14 w/w%,
(2) the content of the polystyrenesulfonate is down to 30 w/w% and up to 80 w/w%,
(3) the void ratio is not less than 20%, and
(4) the water activity is not more than 0.55, or water content is not more than 10 w/w%,

and, furthermore, wherein the preparation completes water absorption within 15 minutes to become a hydrated composition that does not collapse when impregnated with water.

**33.** The preparation of claim 32, wherein the polymeric thickener comprises carboxymethylcellulose and/or a salt thereof.

**34.** The preparation of claim 33, wherein the carboxymethylcellulose salt is carboxymethylcellulose sodium or carboxymethylcellulose calcium.

**35.** The preparation of any of claims 32 to 34, wherein the polystyrenesulfonate is calcium polystyrenesulfonate or sodium polystyrenesulfonate.

**36.** The preparation of any of claims 32 to 35, comprising an edible oil/fat.

**37.** The preparation of any of claims 32 to 36 above, comprising a sugar and/or a sugar alcohol.

**38.** The preparation of claim 37, wherein the sugar and/or the sugar alcohol is one kind or a mixture of two or more kinds selected from the group consisting of white sugar, powdered reduced maltose syrup, sorbitol, and mannitol.

**39.** The preparation of any of claims 32 to 38, comprising a pharmacologically effective ingredient.

**40.** The preparation of claim 39, wherein the polystyrenesulfonate is also a pharmacologically effective ingredient.

**41.** A method of producing the porous dry matrix preparation of any of claims 1 to 10, comprising subjecting a mixed composition obtained by mixing and molding at least a polymeric thickener, an excipient ingredient, and water to thermal drying under normal pressure, wherein the total content of the polymeric thickener and the excipient ingredient is 15 w/w% to 60 w/w%, and wherein the water content before thermal drying is not less than 25 w/w%.

**42.** The method of claim 41, wherein the thermal drying under normal pressure is performed at a temperature of 100°C to 200°C.

**43.** A method of producing the porous dry matrix preparation of any of claims 11 to 19, comprising subjecting a mixed composition obtained by mixing and molding at least a polymeric thickener, polystyrenesulfonate, and water to thermal drying under normal pressure, wherein the total content of the polymeric thickener and the polystyrenesulfonate is 15 w/w% to 60 w/w%, and wherein the water content before thermal drying is not less than 25 w/w%.

44. The method of claim 43, wherein the thermal drying under normal pressure is performed at a temperature of 100˚C to 200˚C.

45. A method of producing the porous dry matrix preparation of any of claims 22 to 31, comprising subjecting a mixed composition obtained by mixing and molding at least a polymeric thickener, an excipient ingredient, and water to thermal drying under normal pressure, wherein the total content of the polymeric thickener and the excipient ingredient is 15 w/w% to 60 w/w%, and wherein the water content before thermal drying is not less than 25 w/w%.

46. The method of claim 45, wherein the thermal drying under normal pressure is performed at a temperature of 100˚C to 200˚C.

47. A method of producing the porous dry matrix preparation of any of claims 32 to 40, comprising subjecting a mixed composition obtained by mixing and molding at least a polymeric thickener, polystyrenesulfonate, and water to thermal drying under normal pressure, wherein the total content of the polymeric thickener and the polystyrenesulfonate is 15 w/w% to 60 w/w%, and wherein the water content before thermal drying is not less than 25 w/w%.

48. The method of claim 47, wherein the thermal drying under normal pressure is performed at a temperature of 100˚C to 200˚C.

49. A method of producing the porous dry matrix preparation of claim 10, comprising subjecting a mixed composition obtained by mixing and molding at least a polymeric thickener, an excipient ingredient, and water to thermal drying under normal pressure to yield a porous dry matrix composition, wherein the total content of the polymeric thickener and the excipient ingredient is 15 w/w% to 60 w/w%, and wherein the water content before thermal drying is not less than 25 w/w%, and
impregnating the dry matrix composition with a solution of a pharmacologically effective ingredient, and, as required, evaporating the solvent for the solution.

50. The method of claim 49, wherein the thermal drying under normal pressure is performed at a temperature of 100˚C to 200˚C.

51. A method of producing the porous dry matrix preparation of claim 31, comprising subjecting a mixed composition obtained by mixing and molding at least a polymeric thickener, an excipient ingredient, and water to thermal drying under normal pressure to yield a porous dry matrix composition, wherein the total content of the polymeric thickener and the excipient ingredient is 15 w/w% to 60 w/w%, and wherein the water content before thermal drying is not less than 25 w/w%, and
impregnating the dry matrix composition with a solution of a pharmacologically effective ingredient, and, as required, evaporating the solvent for the solution.

52. The method of claim 51, wherein the thermal drying under normal pressure is performed at a temperature of 100˚C to 200˚C.

# FIG. 1

# FIG. 2

FIG. 3

**INTERNATIONAL SEARCH REPORT**

| International application No. |
| --- |
| PCT/JP2007/074857 |

A.  CLASSIFICATION OF SUBJECT MATTER
*A61K47/38*(2006.01)i, *A61K9/20*(2006.01)i, *A61K31/795*(2006.01)i, *A61K47/02*
(2006.01)i, *A61K47/10*(2006.01)i, *A61K47/26*(2006.01)i, *A61K47/32*(2006.01)i,
*A61K47/44*(2006.01)i
According to International Patent Classification (IPC) or to both national classification and IPC

B.  FIELDS SEARCHED

Minimum documentation searched (classification system followed by classification symbols)
A61K47/38, A61K9/20, A61K31/795, A61K47/02, A61K47/10, A61K47/26,
A61K47/32, A61K47/44

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho    1971-2008   Toroku Jitsuyo Shinan Koho   1994-2008

Electronic data base consulted during the international search (name of data base and, where practicable, search terms used)
BIOSIS(STN), CAplus(STN), EMBASE(STN), MEDLINE(STN)

C.  DOCUMENTS CONSIDERED TO BE RELEVANT

| Category* | Citation of document, with indication, where appropriate, of the relevant passages | Relevant to claim No. |
| --- | --- | --- |
| X<br><br>Y | JP 10-179053 A  (SAN-EI GEN FFI INC.),<br>07 July, 1998 (07.07.98),<br>Full text; particularly, example 1; Par. No.<br>[0019]<br>& WO 98/08399 A1          & EP 930017 A1<br>& US 2002/039615 A1       & US 6458404 B1<br>& US 2003/077371 A1 | 1,8,9,22,29,<br>30,41,42<br>1-52 |
| X | JP 05-271054 A  (TAKEDA CHEM IND LTD.),<br>19 October, 1993 (19.10.93),<br>Full text; particularly, Claim 18; examples<br>1 to 24<br>& EP 553777 A2           & EP 553777 A3<br>& US 5501861 A           & US 5720974 A<br>& JP 3069458 B2          & JP 2000-264836 A | 1-5,8-10 |

| ☒    Further documents are listed in the continuation of Box C. | ☐    See patent family annex. |
| --- | --- |

*    Special categories of cited documents:
"A"    document defining the general state of the art which is not considered   to
be of particular relevance
"E"    earlier application or patent but published on or after the international filing
date
"L"    document which may throw doubts on priority claim(s) or which is
cited to establish the publication date of another citation or other
special reason (as specified)
"O"    document referring to an oral disclosure, use, exhibition or other means
"P"    document published prior to the international filing date but later than the
priority date claimed

"T"    later document published after the international filing date or priority
date and not in conflict with the application but cited to understand
the principle or theory underlying the invention
"X"    document of particular relevance; the claimed invention cannot be
considered novel or cannot be considered to involve an inventive
step when the document is taken alone
"Y"    document of particular relevance; the claimed invention cannot be
considered to involve an inventive step when the document is
combined with one or more other such documents, such combination
being obvious to a person skilled in the art
"&"    document member of the same patent family

| Date of the actual completion of the international search<br>03 April, 2008 (03.04.08) | Date of mailing of the international search report<br>15 April, 2008 (15.04.08) |
| --- | --- |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2007/074857

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2004-097114 A  (OOKURA SEIYAKU KABUSHIKI KAISHA), 02 April, 2004 (02.04.04), Full text; particularly, Claims; Par. Nos. [0011], [0052] & JP 3835544 B2 | 1-52 |
| Y | WO 99/20285 A1  (SANWA KAGAKU KENKYUSHO CO., LTD.), 29 April, 1999 (29.04.99), Full text; particularly, Claim 1; page 3; preparation example 1 (Family: none) | 1-52 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004099559 A
- JP 2004097114 A
- JP 2004501958 A
- JP 2006349401 A

**Non-patent literature cited in the description**

- *Yakuzaigaku,* 2000, vol. 60 (4), 261-270
- *Yakuri-To-Chiryo,* 1993, vol. 21 (6), 379
- Takozairyo. 1973, 32